# EUROPÄISCHE PATENTSCHRIFT

(11) **EP 3 999 157 B1**
(45) Veröffentlichungstag und Bekanntmachung des Hinweises auf die Patenterteilung: **24.04.2024**
(21) Anmeldenummer: 20716746.1
(22) Anmeldetag: 31.03.2020
(51) Int. Cl.: A61M 16/10, B01D 29/01, B01D 35/30, B01D 46/00, A62B 23/02, A61M 16/00, A61M 16/16, A61M 16/04, A61M 16/08

(54) **BEATMUNGSLEITUNG MIT VERBESSERT AUSTAUSCHBAREM ATEMGAS-FILTER**
BREATHING TUBE WITH A MORE EASILY INTERCHANGEABLE BREATHING-GAS FILTER
CONDUITE DE VENTILATION MUNIE D'UN FILTRE DE GAZ RESPIRATOIRE POUVANT ÊTRE REMPLACÉ DE FAÇON AMÉLIORÉE

(30) Priorität: 19.07.2019 DE 102019119674
(43) Veröffentlichungstag der Anmeldung: 25.05.2022
(60) Teilanmeldung: 23199667.9
(73) Patentinhaber: Hamilton Medical AG, 7402 Bonaduz (CH)
(72) Erfinder: GRUBER, Marco, 7265 Davos Wolfgang (CH); HIDBER, Regula, 7323 Wangs (CH); HUNGER, Jan, 7000 Chur (CH); SCHWAIGER, Ingo, 7000 Chur (CH)
(74) Vertreter: Ruttensperger Lachnit Trossin Gomoll
(86) Internationale Anmeldenummer: PCT/EP2020/059088
(87) Internationale Veröffentlichungsnummer: WO 2021/013388

(56) Entgegenhaltungen:
- WO-A1-2007/095148
- WO-A1-2008/148151
- WO-A1-2011/022779
- WO-A1-2018/074935
- US-A1- 2002 017 085

## Beschreibung

Die vorliegende Erfindung betrifft eine Beatmungsleitung gemäß dem Oberbegriff des Anspruchs 1.

Eine gattungsgemäße Beatmungsleitung ist aus der WO 2018/074935 A1 bekannt.

Eine weitere Beatmungsleitung ist aus der WO 2013/127474 A1 bekannt. Beatmungsleitungen zur Führung von inspiratorischem oder/und exspiratorischem Atemgas werden an Beatmungsvorrichtungen zur wenigstens unterstützenden künstlichen Beatmung von humanen oder tierischen Patienten eingesetzt, um inspiratorisches Atemgas von einer Atemgasquelle zum Patienten zu leiten oder/und um exspiratorisches Atemgas vom Patienten weg zu führen.

In dem Leitungsweg der Beatmungsleitung sind bisweilen Atemgas-Filter angeordnet, um beispielsweise für den Patienten schädliche oder unerwünschte Partikel oder/und Tröpfchen aus dem inspiratorischen Atemgas zu entfernen oder/und um vom Patienten ausgeatmete Krankheitserreger aus dem exspiratorischen Atemgas zu entfernen.

Da naturgemäß ein einmal in Benutzung genommener Atemgas-Filter nur eine begrenzte Lebensdauer aufweist und dann durch einen frischen oder zumindest weniger stark in Anspruch genommen Atemgas-Filter ersetzt werden muss, ist an der bekannten Beatmungsleitung ein Filterträger vorgesehen, welcher einen Austausch eines verbrauchten Atemgas-Filters durch einen ungebrauchten Atemgas-Filter gestattet.

Nachteilig an der aus der WO 2013/127474 A1 bekannten Beatmungsleitung ist, dass der Atemgasfluss für den Wechsel des Atemgas-Filters unterbrochen werden muss, da die Beatmungsleitung für einen Austausch des Atemgas-Filters körperlich unterbrochen oder geöffnet werden muss. Für die Dauer eines Filterwechsels kann somit keine künstliche Beatmung erfolgen.

Auch aus der US 3 556 097 und der GB 1 294 307 A sind jeweils eine Beatmungsleitung mit einem austauschbaren Atemgas-Filter bekannt, bei welchen jeweils der Atemgasfluss für den Filtertausch unterbrochen werden muss.

Es ist daher Aufgabe der vorliegenden Erfindung, die eingangs genannte Beatmungsleitung derart zu verbessern, dass sie einen Wechsel des Atemgas-Filters ohne Unterbrechung eines Atemgasflusses in der Beatmungsleitung gestattet.

Diese Aufgabe wird erfindungsgemäß gelöst durch eine Beatmungsleitung mit allen Merkmalen des Anspruchs 1. Bei dieser Beatmungsleitung weist der Filterträger, wie jener aus der WO 2018/074935 A1 bekannte, eine den Filteraufnahmeraum mit radialem Abstand vom virtuellen Atemgas-Strömungspfad umgebende Wandung mit einer mit radialem Abstand vom virtuellen Atemgas-Strömungspfad angeordneten Einführöffnung auf, durch welche hindurch der Atemgas-Filter in den Filteraufnahmeraum einführbar und in seine betriebsbereite Betriebsposition bringbar ist.

Während bei der Beatmungsleitung der WO 2013/127474 A1 eine Einführöffnung erst durch Trennen zweier Beatmungsleitungsabschnitte im Bereich des Filteraufnahmeraums entsteht, wobei die Einführöffnung durch den virtuellen Atemgas-Strömungspfad durchsetzt ist, also ohne radialen Abstand zum virtuellen Atemgas-Strömungspfad angeordnet ist, kann an der erfindungsgemäßen Beatmungsleitung die radial vom virtuellen Atemgas-Strömungspfad entfernt gelegene Einführöffnung unabhängig vom Betriebszustand der übrigen Komponenten der Beatmungsleitung oder gar der Beatmungsvorrichtung dauerhaft bestehen, sodass ein Atemgas-Filter jederzeit in den Filteraufnahmeraum eingeführt werden kann.

Mit der Einführbewegung kann der Atemgas-Filter ausgehend von der Einführöffnung dem virtuellen Atemgas-Strömungspfad angenähert und so in der Atemgas-Strömung der Beatmungsleitung von dieser durchströmbar betriebsbereit angeordnet werden. In der betriebsbereiten Betriebsposition durchsetzt der virtuelle Atemgas-Strömungspfad den Atemgas-Filter, sodass die Atemgas-Strömung im Bereich des Filteraufnahmeraums auf den Atemgas-Filter trifft und diesen unter Ausnutzung von dessen Filterwirkung durchströmt.

Eine Änderung des Filteraufnahmeraums oder von der den Filteraufnahmeraum umgebenden Wandung ist für das Einführen des Atemgas-Filters in den Filteraufnahmeraum nicht notwendig. Somit kann auch während eines Einführens des Atemgas-Filters in seine Betriebsposition im Filteraufnahmeraum der Beatmungsbetrieb aufrechterhalten werden, an welchem die Beatmungsleitung zum jeweiligen Zeitpunkt beteiligt ist.

Zwar strömt zwischen dem Zeitpunkt einer Entnahme eines verbrauchten Atemgas-Filters und dem Zeitpunkt einer Anordnung eines neuen Atemgas-Filters in dessen Betriebsposition das Atemgas in der Beatmungsleitung ungefiltert. Dies ist jedoch verglichen mit einer vollständigen Unterbrechung einer künstlichen Beatmung ein weitaus geringerer Nachteil.

Grundsätzlich kann daran gedacht sein, dass der Atemgas-Filter lediglich durch die Einführöffnung mit Geschick der jeweils handelnden Bedienperson in den Filteraufnahmeraum und in die Betriebsposition verbracht werden kann. Für einen möglichst schnellen, sicheren und gezielten Filterwechsel mit möglichst kurzer Unterbrechung lediglich der Filterwirkung, nicht jedoch der künstlichen Beatmung an sich, ist es jedoch besonders vorteilhaft, wenn der Filterträger eine Führungsstruktur aufweist, welche eine Einführbewegung des Atemgas-Filters von der Einführöffnung in den Filteraufnahmeraum hinein führt. Somit kann es ausreichen, wenn eine Bedienperson einen neuen Atemgas-Filter lediglich korrekt an der Einführöffnung anordnet und grob in die richtige Richtung antreibt. Die Führungsstruktur am Filterträger sorgt dann dafür, dass der neue Atemgas-Filter eine gewünschte Bewegung in den Filteraufnahmeraum korrekt ausführt. Bevorzugt sorgt die Führungsstruktur nicht nur für eine beliebige Bewegung des Atemgas-Filters in den Filteraufnahmeraum hinein, sondern sorgt in einer bevorzugten Weiterbildung der vorliegenden Erfindung für eine Bewegungsführung des Atemgas-Filters in dessen Betriebsposition.

Die Führungsstruktur kann durch einen den Filterträger durchsetzenden Kanal gebildet sein, der bevorzugt durch den Filteraufnahmeraum umgebende Wandungsabschnitte des Filterträgers gebildet ist. Es kann in einer wegen ihrer Komplexität jedoch weniger bevorzugten Ausführungsform auch daran gedacht sein, dass der Filterträger wenigstens eine Führungsschiene oder/und Führungsnut aufweist, welche die Führungsstruktur bilden.

Bevorzugt ist das Erreichen der Betriebsposition des Atemgas-Filters für eine den Atemgas-Filter, insbesondere manuell, in den Filteraufnahmeraum hinein antreibende Bedienperson wahrnehmbar, beispielsweise durch ein haptisches oder/und akustisches oder/und optisches Signal. Das haptische oder/und akustische Signal kann durch eine Verrastung des Atemgas-Filters in der Betriebsposition erreicht werden. Bevorzugt ist daher im Filteraufnahmeraum, insbesondere an der Führungsstruktur, wenigstens eine Verrastungsformation angeordnet, welche mit einer filterseitigen Verrastungsgegenformation zur Verrastung des Atemgas-Filters in der Betriebsposition wechselwirkt.

Ein optisches Signal kann durch entsprechende Farb- oder/und Formgestaltung des Atemgas-Filters erreicht werden, etwa indem eine Farb- oder/und Formgrenze am Atemgas-Filter relativ zu einem Referenzabschnitt im Bereich des Filteraufnahmeraums, insbesondere im Bereich der Einführöffnung, wie etwa einem Rand der Einführöffnung, eine das Erreichen der Betriebsposition kennzeichnende vorbestimmte Relativstellung einnimmt.

Wenn in der vorliegenden Anmeldung von einem "neuen" Atemgas-Filter die Rede ist, so ist damit nicht notwendigerweise ein fabrikneuer Atemgas-Filter gemeint, wenngleich dies der Regelfall sein wird. Mit einem "neuen" Atemgas-Filter ist jeder Atemgas-Filter bezeichnet, welcher als erster Atemgas-Filter in den Filteraufnahmeraum eingeführt wird oder welcher einen stärker verbrauchten Atemgas-Filter im Filteraufnahmeraum ersetzen soll.

Grundsätzlich kann daran gedacht sein, dass ein verbrauchter Atemgas-Filter unter Umkehrung der Einführbewegung durch die Einführöffnung wieder aus dem Filteraufnahmeraum entnommen wird. Dies ist jedoch wegen des bestehenden Kontaminationsrisikos nicht vorteilhaft. Denn es wird ein möglicherweise kontaminierter verbrauchter Atemgas-Filter durch eine Einführöffnung bewegt, durch welche anschließend ein reiner neuer Filter in den Filteraufnahmeraum eingeführt werden soll. Eine Verringerung dieses Kontaminationsrisikos wird erfindungsgemäß dadurch erreicht, dass die Wandung des Filterträgers eine mit radialem Abstand vom virtuellen Atemgas-Strömungspfad angeordnete, von der Einführöffnung verschiedene Entnahmeöffnung aufweist, durch welche hindurch ein im Filteraufnahmeraum angeordneter Atemgas-Filter aus dem Filteraufnahmeraum entnehmbar ist. Somit kann die Entnahme eines Atemgas-Filters aus dem Filteraufnahmeraum in vorteilhafter Weise durch eine andere Öffnung erfolgen als das Einführen eines Atemgas-Filters in den Filteraufnahmeraum.

Die Entnahme eines verbrauchten Atemgas-Filters, der häufig zu Beginn des Entnahmevorgangs nur mit einem möglicherweise sehr geringen körperlichen Abschnitt für die Einleitung des Entnahmevorgangs zugänglich ist, kann dadurch erleichtert werden, dass die Führungsstruktur zwischen der Einführöffnung und der Entnahmeöffnung angeordnet ist, sodass sie auch eine Entnahmebewegung eines Atemgas-Filters aus dem Filteraufnahmeraum führt. Dies ermöglicht eine besonders vorteilhafte Bedienung, gemäß welcher ein verbrauchter Atemgas-Filter durch den neuen Atemgas-Filter aus dem Filteraufnahmeraum verdrängbar ist. Mit dem Einführen des neuen Atemgas-Filters kann der verbrauchte Atemgas-Filter vom neuen Atemgas-Filter aus dem Filteraufnahmeraum ausgeschoben oder allgemein hinausbewegt werden.

Hierzu weist der neue Atemgas-Filter bevorzugt wenigstens an seinem in Einführrichtung vorauseilenden Endbereich eine Verstärkungsformation, wie etwa eine verstärkende Randleiste, auf, um auf den im Filteraufnahmebereich angeordneten verbrauchten Atemgas-Filter ausreichend Kraft für dessen Verdrängung ausüben zu können.

Da zur Vermeidung von Fehlern im Beatmungsbetrieb bevorzugt baugleiche Atemgas-Filter in zeitlicher Aufeinanderfolge verwendet werden, weist der Atemgas-Filter auch an seinem in Einführrichtung nachlaufenden Endbereich eine Verstärkungsformation, wie etwa eine verstärkende Randleiste, auf, um der verdrängenden Kraft des neuen Atemgas-Filters bei dessen Einführung standhalten zu können. Denn Ziel der Kraftübertragung zwischen neuem unverbrauchtem Atemgas-Filter ist nicht die Verformung wenigstens eines der beiden Filter, sondern deren gewünschte Bewegung. Bevorzugt weist daher ein Atemgas-Filter ein von Atemgas durchströmbares Filtermaterial, insbesondere ebenes Filtermaterial, auf, welches von einem im Vergleich zum Filtermaterial starren Rahmen umgeben ist. Der Rahmen weist bevorzugt als umlaufender Rahmen eine zentrale Durchgangsöffnung auf, durch welche hindurch das Atemgas im Beatmungsbetrieb das vom Rahmen umgebene Filtermaterial durchströmt.

Wenngleich grundsätzlich komplexe Bewegungswege zum Einführen oder/und zum Entnehmen eines Atemgas-Filters denkbar oder zur Vermeidung einer unerwünschten Entfernung eines Atemgas-Filters aus seiner Betriebsposition sogar vorteilhaft sind, können komplexe Bewegungswege hohe Verlagerungskräfte erfordern, um die gewünschte Bewegung zu bewirken. Ein Filteraustausch kann dann mit vorteilhaft geringer Verlagerungskraft ausgeführt werden, wenn der Atemgas-Filter längs einer durch die Führungsstruktur definierten knickfreien Führungsbahn durch die Einführöffnung hindurch, auf den virtuellen Atemgas-Strömungspfad zu, in den Filteraufnahmeraum hinein und in die Betriebsposition sowie aus der Betriebsposition, vom virtuellen Atemgas-Strömungspfad weg, aus dem Filteraufnahmeraum hinaus durch die Entnahmeöffnung hindurch bewegbar ist. Bevorzugt ist diese knickfreie Führungsbahn eine geradlinige Führungsbahn. Dann können Einführöffnung und Entnahmeöffnung an längs der Führungsbahn entgegengesetzten Seiten der Wandung des Filteraufnahmeraums ausgebildet sein.

Um das oben angesprochene Kontaminationsrisiko zu verringern, kann der Filterträger dazu ausgebildet sein, eine Bewegung eines Atemgas-Filters in den Filteraufnahmeraum hinein und aus diesem hinaus nur in genau einer Richtung zuzulassen. Dies gilt insbesondere für die Führungsstruktur, durch welche die Bewegbarkeit des Atemgas-Filters in den Filterträger hinein und aus diesem hinaus besonders effektiv beeinflussbar ist. Zu diesem Zweck kann der Filterträger, und am Filterträger insbesondere die Führungsstruktur, eine Hemmungsformation aufweisen, welche eine Bewegung des Atemgas-Filters durch die Einführöffnung in den Filteraufnahmeraum hinein zulässt und eine entgegengesetzt gerichtete Bewegung des Atemgas-Filters durch die Einführöffnung hemmt. Die Hemmungsformation kann in der Art eines Klinkengesperres oder Rastgesperres ausgebildet sein.

Grundsätzlich kann die Hemmungsformation durch Einwirkung unmittelbar auf poröses Filtermaterial oder ein entsprechend verformbares Rahmenmaterial eines oben genannten Rahmens die genannte Hemmungswirkung erzeugen. Beispielsweise kann eine in Richtung der zugelassenen Bewegung geneigte Verzahnungsausbildung am Filterträger bzw. an der Führungsstruktur als Hemmungsformation vorgesehen sein, sodass das Filter- oder Rahmenmaterial bei Bewegung in der zugelassenen Bewegungsrichtung unter der Verzahnungsausbildung hinweggleitet und die Verzahnungsausbildung beim Versuch einer Bewegung des Atemgas-Filters in die gehemmte Bewegungsrichtung in das Filter- oder Rahmenmaterial eindringt.

Eine besonders sichere unidirektionale Bewegungshemmung kann dadurch erreicht werden, dass der Atemgas-Filter eine Hemmungsgegenformation aufweist, welche mit der Hemmungsformation des Filterträgers zur Erzielung der unidirektionalen Bewegbarkeit des Atemgas-Filters zusammenwirkt. Bevorzugt wirkt die Hemmungsgegenformation mit der Hemmungsformation formschlüssig zusammen. Durch Ausbildung einer Hemmungsgegenformation, die mit der am Filterträger bzw. an der Führungsstruktur vorgesehenen Hemmungsformation zusammenwirken muss, kann außerdem das Risiko einer Anordnung eines falschen Atemgas-Filters in einem Filterträger erheblich vermindert werden. Bei abweichender oder fehlender Hemmungsgegenformation wird das Einführen eines falschen neuen Atemgas-Filters für die jeweils ausführende Bedienperson sofort wahrnehmbar.

In einer besonders effektiven und daher bevorzugten Ausgestaltung bilden die Hemmungsformation und die Hemmungsgegenformation eine Mehrzahl von längs der zugelassenen Bewegungsrichtung hintereinander gelegenen unidirektional überwindbaren Verrastungen. Beispielsweise können die Hemmungsformation und die Hemmungsgegenformation jeweils eine aus ausreichend elastischem Material gebildete, längs der Führungsbahn verlaufende Sägezahnformation aufweisen. Die bezüglich der Führungsbahn bzw. der Bewegungsrichtung weniger stark geneigten Schrägflächen der beiden Sägezahnformationen können in einer Richtung aneinander vorbei gleiten, während in der entgegengesetzten Richtung die bezüglich der Führungsbahn Beziehung weise der Bewegungsrichtung stärker geneigten Schrägflächen der beiden Sägezahnformationen eine zerstörungsfrei nicht überwindbare körperliche Bewegungsbarriere bilden.

Die oben genannten Verstärkungsformationen, insbesondere Randleisten, besonders bevorzugt der umlaufende Rahmen, sind bzw. ist bevorzugt gasundurchlässig, sodass der Atemgas-Filter eine definierte Durchgangsöffnung für eine Durchströmung mit Atemgas bereitstellt. Bevorzugt dient der oben genannte Rahmen oder eine verstärkende Randleiste des Atemgas-Filters in der Betriebsposition des Atemgas-Filters zur Abdichtung der Einführöffnung. Weiter bevorzugt dient der oben genannte Rahmen oder eine verstärkende Randleiste des Atemgas-Filters in der Betriebsposition des Atemgas-Filters auch zur Abdichtung der Entnahmeöffnung. Somit kann lediglich durch Anordnen des Atemgas-Filters in dessen Betriebsposition im Filterträger bzw. im Filteraufnahmeraum sichergestellt sein, dass der von Atemgas durchströmte Filteraufnahmeraum im Beatmungsbetrieb von der den Filterträger umgebenden Umgebungsatmosphäre getrennt ist. Am Randbereich der Einführöffnung oder/und der Entnahmeöffnung kann gemäß einer bevorzugten Weiterbildung der vorliegenden Erfindung eine Dichtlippe oder allgemein eine sonstige Dichtungsformation vorgesehen sein, welche in der Betriebsposition des Atemgas-Filters an der verstärkenden Randleiste oder am Rahmen des Atemgas-Filters dichtend anliegt. Wie in diesem Abschnitt beschrieben ist, kann der Rahmen oder wenigstens die Randleiste eine Strömungsbarriere gegen eine unerwünschte Durchströmung der Einführöffnung oder/und der Entnahmeöffnung mit Atemgas bilden. Hierfür ist es jedoch erforderlich, dass die die Strömungsbarriere bildende Randleiste bzw. Rahmen gasundurchlässig ausgebildet sind.

Die die Einführöffnung oder/und die Entnahmeöffnung aufweisende Wandung kann Teil eines den Filteraufnahmeraum im betriebsbereiten Zustand umgebenden Gehäuses des Filterträgers sein. Zu Zwecken der Reinigung oder/und der Wartung kann das Gehäuse als teilbares Gehäuse wenigstens zwei voneinander entfernbare und aneinander annäherbare Gehäuseteile aufweisen. Es soll also nicht ausgeschlossen sein, dass das Gehäuse des Filterträgers in wenigstens zwei Teile zerlegbar ist. Offenbarungsgemäß soll lediglich ein solches Zerlegen für einen Atemgas-Filterwechsel nicht notwendig sein.

Grundsätzlich muss der Filterträger von Atemgas durchströmbar sein. Hierzu kann der Filterträger beiderseits des Filteraufnahmeraums strömungsführende Leitungsabschnitte aufweisen, etwa Anschlussstutzen für den jeweiligen Anschluss eines Beatmungsschlauches daran. Häufig ist es erwünscht, die Temperatur des Atemgas-Filters in dessen Betriebsposition beeinflussen zu können, insbesondere den Atemgas-Filter in seiner Betriebsposition beheizen zu können. Für die Anordnung einer Filterheizung am Filterträger können fluchtende Leitungsabschnitte beiderseits des Filteraufnahmeraums nachteilig sein, da sie eine Heizfläche einer am Filterträger lösbar anordenbaren Heizvorrichtung beschränken können. Zur Erzielung einer möglichst großen Heizfläche bei gleichzeitiger Trennbarkeit des Filterträgers von einer die Heizleistung liefernden Heizvorrichtung ist es vielmehr vorteilhaft, wenn der Filterträger beiderseits des Filteraufnahmeraums strömungsführende Leitungsabschnitte aufweist, deren jeweils zugeordnete Abschnitte des virtuellen Atemgas-Strömungspfads miteinander einen Winkel einschließen.

Zur Beeinflussung der Temperatur des Atemgas-Filters kann die Beatmungsleitung eine Filterheizung als die oben genannte Heizvorrichtung aufweisen, welche den Filterträger im betriebsbereiten Zustand wenigstens abschnittsweise umgibt.

Um den Filterträger von der Filterheizung zur Reparatur oder/und Wartung entfernen zu können, kann die Filterheizung eine als vom Filterträger gesondertes Bauteil ausgebildete Filterträgeraufnahme aufweisen, in welche der Filterträger einsetzbar ist und aus welcher ein eingesetzter Filterträger entnehmbar ist. Der Filterträger kann dabei von der Filterheizung entfernt werden, ohne dass hierfür der Atemgasstrom unterbrochen oder die Beatmungsleitung geöffnet werden muss

Dabei kann ein Filteraustausch selbst dann durchgeführt werden, wenn der Filterträger in der Filterträgeraufnahme der Filterheizung eingesetzt ist, wenn gemäß einer vorteilhaften Weiterbildung der vorliegenden Erfindung die Filterheizung eine Wandung mit einer Durchlassöffnung aufweist, welche im betriebsbereiten Zustand mit der Einführöffnung derart fluchtet, dass ein Atemgas-Filter durch die Durchlassöffnung in die Einführöffnung und damit in den Filterträger einführbar ist. Entsprechendes gilt mutatis mutandis auch für die Entnahmeöffnung. Eine Wandung der Filterheizung sollte gemäß dieser vorteilhaften Weiterbildung folglich eine Durchführungsöffnung aufweisen, welche im betriebsbereiten Zustand mit der Entnahmeöffnung fluchtet.

Grundsätzlich kann der Atemgas-Filter bis zu seiner Verwendung in beliebiger Weise aufbewahrt und an den Filterträger herangeführt werden. Bevorzugt ist der Atemgas-Filter bis kurz vor seiner Einführung in den Filteraufnahmeraum verpackt, um den Atemgas-Filter vor äußeren Einflüssen, insbesondere vor Verschmutzung zu schützen. Wie oben bereits dargelegt wurde, ist der Atemgas-Filter längs eines Einführbewegungswegs in den Filteraufnahmeraum einführbar. Der Einführbewegungsweg ist bevorzugt die oben genannte durch die Führungsstruktur definierte Führungsbahn. Zum Schutz vor äußeren Einflüssen ist der Atemgas-Filter in einem Ausgangszustand vor seiner Einführung in den Filteraufnahmeraum bevorzugt in einer Hülle aufgenommen ist, wobei die Hülle weiter bevorzugt dazu ausgebildet ist, einen Beitrag zur Einführung des Atemgas-Filters in den Filteraufnahmeraum zu leisten.

Um den Atemgas-Filter nicht nur während seiner Lagerung und seines Transports vor äußeren Einflüssen zu schützen, sondern um den Atemgas-Filter unter möglichst hygienischen Bedingungen in den Filteraufnahmeraum einführen zu können, weist die Hülle bevorzugt eine Kopplungsformation auf. Ebenso weist entweder der Filterträger oder die Filterheizung, je nachdem, ob der neue Atemgas-Filter unmittelbar durch die Einführöffnung des Filterträgers oder zuvor durch eine Durchlassöffnung einer Filterheizung hindurch in den Filteraufnahmeraum eingeführt wird, eine mit der Kopplungsformation koppelbare Kopplungsgegenformation auf.

Gemäß einer bevorzugten Weiterbildung der vorliegenden Erfindung ist die Kopplungsformation mit der Kopplungsgegenformation des Filterträgers oder der Filterheizung derart koppelbar, dass in einem Übergabezustand, in welchem eine zur Ausgabe des Atemgas-Filters aus der Hülle ausgebildete Zugangsöffnung der Hülle mit der Einführöffnung längs des Einführbewegungswegs fluchtet, die Hülle an der Kopplungsgegenformation gehalten ist. So kann nicht nur die Hülle mit dem neuen Atemgas-Filter am Filterträger oder an der Filterheizung zum vorübergehenden Verbleib angeordnet werden, ohne dass sie fortwährend gehalten werden muss, sondern die Hülle kann durch die Eingriffswechselwirkung von Kopplungsformation und Kopplungsgegenformation wenigstens im Übergabezustand die Einführöffnung und gegebenenfalls auch die Durchlassöffnung abdecken. Dadurch ist auch der Filteraufnahmeraum durch die über ihre Kopplungsformation an den Filterträger oder an die Filterheizung angekoppelte Hülle vor äußeren Einflüssen, wie Verschmutzung, geschützt. Dann, wenn die Hülle mittels ihrer Kopplungsformation mit der Kopplungsgegenformation gekoppelt ist, kann der Filterträger bequem bewegt werden, ohne dass hierdurch die Hülle vom Filterträger bzw. von der Filterheizung gelöst wird. Dies vereinfacht einen Filtertausch erheblich.

Da im Übergabezustand die Zugangsöffnung der Hülle mit der Einführöffnung und gegebenenfalls auch mit der Durchlassöffnung längs des Einführbewegungswegs fluchtet, kann der neue, noch in der Hülle angeordnete Atemgas-Filter besonders einfach von der Hülle durch die genannte wenigstens eine Öffnung in den Filteraufnahmeraum verbracht werden. Dies kann hygienisch besonders vorteilhaft ohne Berührung des Atemgas-Filters durch Personal sowie ohne Kontakt des Atemgas-Filters mit der Außenumgebung erfolgen, wenn wenigstens ein Wandabschnitt der Hülle relativ zur Kopplungsformation derart verlagerbar ist, dass der Atemgas-Filter durch Verlagerung des verlagerbaren Wandabschnitts relativ zur Kopplungsformation durch die Zugangsöffnung aus der Hülle ausschiebbar ist.

Grundsätzlich kann der verlagerbare Wandabschnitt an der übrigen Hülle ähnlich einem verlagerbaren Kolben in einem Zylinder ausgestaltet sein, sodass der verlagerbare Wandabschnitt in Anlageeingriff mit dem Atemgas-Filter, genauer mit dessen verstärkender Randleiste, stärker bevorzugt mit dessen das Filtermaterial umgebendem Rahmen, gebracht werden kann und durch Bewegung des Wandabschnitts aus der Zugangsöffnung ausgeschoben werden kann. In diesem Fall befindet sich der Atemgas-Filter im Aufbewahrungszustand einer den Atemgas-Filter und die Hülle umfassenden Atemgas-Filteranordnung zwischen dem verlagerbaren Wandabschnitt und der Zugangsöffnung. Ein derart aufwendiger verlagerbarer Wandabschnitt ist zwar grundsätzlich möglich, jedoch aufgrund der Komplexität dieser Ausgestaltung nicht bevorzugt. Aus Gründen einer möglichst einfachen Herstellung und Handhabung, und damit auch aus Gründen möglichst niedriger Kosten, ist bevorzugt, dass die Hülle wenigstens abschnittsweise derart flexibel ausgebildet ist, dass die den Atemgas-Filter durch die Zugangsöffnung aus der Hülle ausschiebende Verlagerung eine Verformung des verlagerbaren Wandabschnitts ist. Die Hülle kann zumindest in ihrem durch Verformung verlagerbaren Wandabschnitt aus weichelastischem Kunststoff, wie beispielsweise aus einem Polyolefin oder auch aus besonders reißfestem Polyethylenterephthalat, insbesondere aus einer formlabilen Kunststofffolie gebildet sein. Die Hülle kann jedoch immer noch mit geringer Kraft verformbar mit größeren Wandstärken aus einem Elastomer, wie beispielsweise Gummi oder Silikon, hergestellt sein. Die Hülle kann wenigstens abschnittsweise als Faltenbalg ausgebildet sein, welcher in Richtung auf die Zugangsöffnung zu und von dieser weg verformbar ist. Im Fall einer wenigstens abschnittsweisen Ausbildung der Hülle als Faltenbalg kann die Hülle auch aus einem steiferem Material gebildet sein, welches sich als ebene Außenfläche mit gleicher Dicke wie eine durch Handangriff leicht verformbare Elastomerhülle nur mit erheblich größerer Kraft oder durch werkzeugfreien Handangriff nicht mehr verformen lässt.

Wiederum ist die Hülle zur effektiven Einführung des Atemgas-Filters aus einem Innenraum der Hülle heraus, durch die Einführöffnung hindurch, in den Filteraufnahmeraum des Filterträgers hinein, bevorzugt wenigstens an ihrem von der Kopplungsformation fernliegenden Ende verformbar ausgeführt. Die Hülle kann von diesem fernliegenden Ende bis zu ihrer Zugangsöffnung verformbar ausgeführt sein. Mit "verformbar" ist dabei eine werkzeugfreie Verformbarkeit durch Handangriff selbst einer schwachen Person, wie etwa einer untrainierten Seniorin, bezeichnet.

Wenn oben ausgesagt ist, dass die Atemgas-Filteranordnung, also die Hülle mit darin aufgenommenem Atemgas-Filter, durch die Kopplungsformation der Hülle im Übergabezustand mit der Kopplungsgegenformation in der oben beschriebenen Weise gekoppelt ist, soll dies nicht bedeuten, dass die Atemgas-Filteranordnung nur im Übergabezustand mit der Kopplungsgegenformation gekoppelt oder koppelbar ist, wenngleich dies nicht ausgeschlossen sein soll. Bevorzugt ermöglichen die Kopplungsformation und die Kopplungsgegenformation bereits einen Kopplungseingriff vor Erreichen des Übergabezustands, wodurch die Hülle bzw. die Atemgas-Filteranordnung bei hergestelltem Kopplungseingriff relativ zum Filterträger längs einer Kopplungsbahn in eine dem Übergabezustand entsprechenden Relativstellung verlagerbar ist. Damit die Hülle bzw. die Atemgas-Filteranordnung im Übergabezustand mit einer ausreichenden Haltekraft durch den Kopplungseingriff im Übergabezustand gehalten werden kann, verläuft die Kopplungsbahn bevorzugt quer zum Einführbewegungsweg des Atemgas-Filters, wenn dieser in den Filterträger hinein eingeführt wird. Dadurch kann der Kopplungseingriff von Kopplungsformation und Kopplungsgegenformation längs des Einführbewegungswegs eine Abhebesicherung, wegen ihrer großen Sicherheit bevorzugt eine formschlüssige Abhebesicherung, bilden. Bevorzugt ist daher der Kopplungseingriff von Kopplungsformation und Kopplungsgegenformation ein formschlüssiger Kopplungseingriff.

Die Kopplungsformation kann gesondert von der übrigen Hülle ausgebildet sein und mit dieser verbunden sein. Dies ist bevorzugt, wenn eine den Atemgas-Filter umgebende Haut der Hülle aus einer formlabilen Kunststofffolie gebildet ist. Dann kann die Kopplungsformation als eigensteifes Bauteil ausgebildet und, beispielsweise durch Verkleben oder Verschweißen, mit der Haut verbunden sein.

Dann, wenn die Haut der Hülle ausreichend formstabil ist, um während eines Filtertauschs im Bereich des Kopplungseingriffs erwartbare Haltekräfte aufzunehmen, kann die Kopplungsformation einstückig mit der Haut der Hülle ausgebildet sein.

Wenngleich nicht ausgeschlossen sein soll, dass die Hülle bzw. die Atemgas-Filteranordnung ausgehend von einem anfänglichen Kopplungseingriff teilweise oder vollständig rotatorisch um eine Drehachse in den Übergabezustand gedreht wird, ist es bevorzugt, wenn die Hülle bzw. Atemgas-Filteranordnung nach Herstellung eines anfänglichen Kopplungseingriffs durch eine leicht und sicher führbare translatorische Verschiebebewegung in den Übergabezustand verbracht werden kann. Zur einfachen Handhabung ist die Hülle bzw. Atemgas-Filteranordnung bevorzugt nur durch eine translatorische Bewegung in den Übergabezustand verschiebbar.

Zur Führung der Bewegung der Hülle bzw. der Atemgas-Filteranordnung längs ihrer Kopplungsbahn bei hergestelltem Kopplungseingriff zwischen Kopplungsformation und Kopplungsgegenformation kann eine Formation aus Kopplungsformation und Kopplungsgegenformation wenigstens einen Vorsprung umfassen und kann weiterhin die jeweils andere Formation aus Kopplungsformation und Kopplungsgegenformation wenigstens eine Nut aufweisen, wobei der wenigstens eine Vorsprung wenigstens in dem Übergabezustand, bevorzugt schon während der Relativbewegung, längs der Kopplungsbahn in die wenigstens eine Nut eingreift. So kann eine einen Vorsprung in Umfangsrichtung um die virtuelle Kopplungsbahn umgreifende Nut zum einen die Bewegung der Hülle bzw. der Atemgas-Filteranordnung längs der Kopplungsbahn führen. Hierzu verläuft die Nut längs der Kopplungsbahn. Zum anderen kann die den Vorsprung umgreifende Nut die Hülle bzw. die Atemgas-Filteranordnung gegen ein Abheben orthogonal zur Kopplungsbahn vom Filterträger weg sichern.

Vorzugsweise weist die Hülle wenigstens zwei oder bevorzugt genau zwei Vorsprünge oder Nuten als die Kopplungsformation auf, und zwar zur Bereitstellung einer Kippsicherung der mit der Kopplungsgegenformation gekoppelten Hülle bevorzugt auf jeder Seite der Zugangsöffnung je wenigstens einen Vorsprung oder wenigstens eine Nut oder besonders bevorzugt je genau einen Vorsprung oder genau eine Nut. Die Ausbildung von Nuten in Seitenflächen der Hülle ermöglicht eine Ausbildung der Hülle mit wenigstens zwei parallelen Seitenflächen mit jeweils ebener Schmiegefläche, die frei sind von einem Überstand über die Seitenflächen, sodass sich die Hüllen bzw. die Atemgas-Filteranordnungen in einer Richtung orthogonal zu diesen parallelen ebenen Seitenflächen effizient stapeln lassen.

Alternativ kann die Kopplungsformation wenigstens einen, vorzugsweise zwei Vorsprünge umfassen, wobei wiederum besonders bevorzugt auf jeder Seite der Zugangsöffnung je wenigstens ein Vorsprung ausgebildet ist. Der Vorsprung, der bevorzugt in Richtung von der Zugangsöffnung weg von der Haut der Hülle vorsteht, kann der bevorzugt flexibel ausgebildeten Hülle im Bereich der Zugangsöffnung eine erhöhte Formstabilität verleihen.

Unabhängig von der Ausbildung der Kopplungsformation als wenigstens eine Nut oder/und als wenigstens ein Vorsprung erstreckt sich die wenigstens eine Nut oder/und der wenigstens eine Vorsprung zur vorteilhaften Abdichtung der Hülle gegenüber der Filterheizung oder dem Filterträger im Übergabezustand längs der Kopplungsbahn über die gesamte Länge der Zugangsöffnung.

Üblicherweise sind die Atemgas-Filter flächige Gebilde, also Gegenstände, welche in zwei zueinander orthogonalen Raumrichtungen eine wesentlich größere Abmessung aufweisen als in einer zu jeder der beiden orthogonalen Raumrichtungen orthogonalen Dickenrichtung. Die Hülle weist zur effizienten Raumausnutzung bevorzugt eine dem Atemgas-Filter folgende Gestalt auf, sodass auch die Hülle in zwei zueinander orthogonalen Raumrichtungen eine wesentlich größere Abmessung aufweist als in einer zu jeder der beiden zueinander orthogonalen Raumrichtungen orthogonalen Dickenrichtung.

Zur Vermeidung von Fehlanordnungen sind bevorzugt sowohl die Hülle als auch der darin aufgenommene Atemgas-Filter bezüglich einer zur Dickenrichtung orthogonalen Spiegelsymmetrieebene spiegelsymmetrisch oder bezüglich einer zur Dickenrichtung und zur Zugangsöffnungsfläche orthogonalen Invarianzachse bezüglich einer Rotation um 180° gestaltinvariant. Somit kommt es bevorzugt auf eine Durchströmungsrichtung des Atemgas-Filters nicht an. Er kann bevorzugt in jeder von zwei möglichen entgegengesetzten Durchströmungsrichtungen durchströmt werden.

Ein besonderer Vorteil der Einführung eines Atemgas-Filters in den Filteraufnahmeraum aus einer Hülle heraus liegt in der Möglichkeit, die Hülle wenigstens im Übergabezustand gegenüber dem Filterträger oder gegenüber der Filterheizung abzudichten, sodass während des Filtertauschs ein in der Beatmungsleitung herrschender Atemgasdruck zum Großteil oder sogar vollständig aufrechterhalten werden kann. Dies gilt insbesondere für den während einer Beatmung wichtigen end-exspiratorischen Überdruck (PEEP). Daher ist gemäß einer vorteilhaften Weiterbildung der vorliegenden Beatmungsleitung oder/und der Atemgas-Filteranordnung vorgesehen, dass wenigstens eine Formation aus Kopplungsformation und Kopplungsgegenformation eine Dichtungsstruktur aufweist, welche wenigstens im Übergabezustand dichtend an der jeweils anderen Struktur anliegt.

Es sei jedoch an dieser Stelle darauf hingewiesen, dass auch ohne gesonderte Dichtungsstruktur zwischen Kopplungsformation und Kopplungsgegenformation bestehende Spalte für die Dauer des Filtertauschs eine ausreichende Abdichtung und damit Aufrechterhaltung eines Atemgasdrucks in der Beatmungsleitung bewirken können. Dies gilt insbesondere dann, wenn ein solcher Spalt, ähnlich einer Labyrinthdichtung, in seinem Verlauf von einem ersten Raum zu einem vom ersten zu trennenden zweiten Raum mehrfach abgewinkelt ist.

Bevorzugt ist die Dichtungsstruktur an der Kopplungsgegenformation ausgebildet oder angeordnet, da dann die Kopplungsgegenformation an einem formstabileren Gegenstand, wie etwa einem Gehäuse einer Filterheizung oder am Filterträger, ausgebildet werden kann und so eine von der Kopplungsgegenformation getragene Dichtungsstruktur durch einen vorteilhaft steifen und stabilen Untergrund abgestützt sein kann. Bevorzugt ist die Dichtungsstruktur ein Dichtungsstreifen, besonders bevorzugt mit einer von dem die Dichtungsstruktur tragenden Untergrund weg abstehenden Dichtlippe. Der Dichtungsstreifen erstreckt sich bevorzugt längs der Kopplungsbahn. Um im Übergabezustand die Hülle mit der Zugangsöffnung gegen entweder die Filterheizung mit der Durchlassöffnung oder den Filterträger mit der Einführöffnung möglichst wirksam abzudichten, verläuft an der Filterheizung oder am Filterträger je wenigstens ein Dichtungsstreifen beiderseits der Durchlassöffnung bzw. der Einführöffnung, und zwar über eine Länge, welche wenigstens der Länge der Zugangsöffnung längs der Kopplungsbahn entspricht, vorzugsweise größer ist als die Länge der Zugangsöffnung längs der Kopplungsbahn. Aus Gründen einer möglichst wirksamen Abdichtung ist jeder der Dichtungsstreifen vorzugsweise länger als die längs der Kopplungsbahn längere der beiden im Übergabezustand einander gegenüberliegenden Öffnungen aus Zugangsöffnung und Durchlassöffnung oder aus Zugangsöffnung und Einführöffnung.

Bevorzugt ist die Dichtungsstruktur aus einem weniger steifen Material hergestellt als die sie tragende Formation aus Kopplungsformation und Kopplungsgegenformation. Als Dichtungsstruktur gilt auch eine Verformungsformation an einer Nut, wobei die Verformungsformation derart ausgebildet ist, dass sie an einem in die Nut eingeführten Vorsprung den Vorsprung verformend und damit dichtend anliegt. Dies gilt insbesondere für den Fall, dass der Vorsprung aus einem grundsätzlich auch für Dichtungsaufgaben geeigneten Elastomer, wie etwa Gummi oder Silikon, gebildet ist. Dann kann die als Verformungsformation ausgebildete Dichtungsstruktur aus demselben Material oder sogar aus einem steiferen Material als die sie tragende Formation gebildet sein. Selbstverständlich gilt dies mutatis mutandis auch für eine als Verformungsformation am Vorsprung ausgebildete Dichtungsstruktur, welche dazu dient, einen Abschnitt der Nut zu verformen.

Eine Verformungsformation als Dichtungsstruktur kann in Richtung von der sie tragenden Formation weg sich verjüngend ausgebildet sein, um durch höhere Flächenpressung an ihrem von der Formation fernliegenden Ende eine Verformung des an ihr anliegenden Bauteilabschnitts zu erleichtern. Bevorzugt weist die Dichtungsstruktur, dies gilt auch für die Verformungsformation, längs der Kopplungsbahn wenigstens in einem Bereich, in welchem die Dichtungsstruktur längs der Kopplungsbahn neben einer der sich im Übergabezustand gegenüberliegenden Öffnungen verläuft, wenigstens abschnittsweise, vorzugsweise vollständig, einen konstanten Querschnitt auf.

Die oben genannten Vorteile sind oben anhand der Einführung eines neuen Atemgas-Filters in den Filteraufnahmeraum beschrieben. Sie betreffen jedoch ebenso die Entnahme eines gebrauchten Atemgas-Filters aus dem Filteraufnahmeraum. Daher ist bevorzugt vorgesehen, dass der Atemgas-Filter längs eines Entnahmebewegungswegs aus dem Filteraufnahmeraum entnehmbar ist, wobei der Filterträger oder die Filterheizung eine weitere Kopplungsgegenformation aufweist, wobei eine Hülle derart mit der weiteren Kopplungsgegenformation koppelbar ist, dass in einem Ausgabezustand, in welchem die Zugangsöffnung der Hülle mit der Entnahmeöffnung längs des Entnahmebewegungswegs fluchtet, die Hülle an der weiteren Kopplungsgegenformation gehalten ist. Bevorzugt ist der Entnahmebewegungsweg die oben genannte durch die Führungsstruktur definierte Führungsbahn.

Eine leere Hülle, die nach dem Einführen des Atemgas-Filters aus der Hülle in den Filteraufnahmeraum übrig bleibt, kann somit aufbewahrt werden und beim nächsten Filtertausch als Aufnahme-Hülle vor dem Ausschieben des gebrauchten Atemgas-Filters durch den neuen Atemgas-Filter mit ihrer Kopplungsformation mit der weiteren Kopplungsgegenformation gekoppelt werden. Dann kann der gebrauchte Atemgas-Filter - unzugänglich für das den Filtertausch ausführende Personal - in die Aufnahme-Hülle ausgeschoben werden.

Die weitere Kopplungsgegenformation ist bevorzugt wie die oben beschriebene Kopplungsgegenformation ausgebildet, sodass sie in gleicher Weise mit der Kopplungsformation der Hülle zusammenwirken kann wie die Kopplungsgegenformation. Oben beschriebene Weiterbildungen der Kopplungsgegenformation sind daher auch Weiterbildungen der weiteren Kopplungsgegenformation.

Das Einführen eines neuen Atemgas-Filters aus einer im Kopplungseingriff mit der Kopplungsgegenformation stehenden Atemgas-Filteranordnung und ein dadurch bewirktes Ausschieben eines verbrauchten Atemgas-Filters aus der Betriebsposition in eine mit der weiteren Kopplungsgegenformation in Kopplungseingriff stehenden Aufnahme-Hülle ermöglicht eine besonders vorteilhafte dauerhafte Aufrechterhaltung des in der Beatmungsleitung herrschenden Atemgasdrucks. Dadurch kann der Filtertausch nahezu ohne Beeinträchtigung der Beatmungssituation eines an die Beatmungsleitung angeschlossen Patienten bei laufender künstlicher Beatmung durchgeführt werden.

Wie oben bereits beschrieben wurde, weist der Atemgas-Filter bevorzugt einen ein Filtermaterial umgebenden gasundurchlässigen Rahmen auf. Dieser Rahmen kann ein im Rahmen aufgenommenes, zentral zugängliches Filtermaterial zuverlässig tragen und im Atemgasstrom halten. Eine vorteilhaft effiziente dauerhafte Durchströmung des Filtermaterials mit Atemgas unter größtmöglicher Vermeidung von Leckageströmen aus dem Filteraufnahmeraum hinaus kann dadurch erreicht werden, dass mit Abstand von der Atemgas-Strömungsbahn, bevorzugt zwischen der Atemgas-Strömungsbahn und der Einführöffnung oder/und der Entnahmeöffnung, stärker bevorzugt am Randbereich der Einführöffnung oder/und der Entnahmeöffnung, eine Dichtungsformation, insbesondere eine Dichtlippe, vorgesehen ist, welche in der Betriebsposition des Atemgas-Filters am Rahmen des Atemgas-Filters dichtend anliegt. Zur Erzielung einer möglichst umfassenden, lange anhaltenden Dichtungswirkung läuft die Dichtungsformation bevorzugt geschlossen um die Atemgas-Strömungsbahn um, wobei sich der vom Atemgasstrom zugängliche Teil des Filtermaterials des Atemgas-Filters bevorzugt vollständig radial innerhalb des von der Dichtungsformation eingeschlossenen Bereichs befindet.

Grundsätzlich kann diese Dichtungsformation auch am Rahmen des Atemgas-Filters angeordnet oder/und ausgebildet sein. Jedoch ist eine möglichst glatte zur Durchströmungsrichtung des Filtermaterials orthogonale Grenzfläche des Rahmens zur vereinfachten Stapelung auch größerer Mengen an Atemgas-Filtern bzw. Atemgas-Filteranordnungen bevorzugt.

Die vorliegende Anmeldung beschreibt außerdem eine Atemgas-Filteranordnung, wie sie oben bereits beschrieben wurde und wie sie grundsätzlich aus der WO 2007/095148 A1 bekannt ist, umfassend einen in einer Hülle aufgenommenen Atemgas-Filter, wobei die Hülle eine Kopplungsformation zur Kopplung mit einer Kopplungsgegenformation eines Filterträgers oder einer Filterheizung sowie eine Zugangsöffnung zur Ausgabe des Atemgas-Filters aus der Hülle aufweist. Wenigstens ein Wandabschnitt der Hülle ist relativ zur Kopplungsformation derart verlagerbar, dass der Atemgas-Filter durch Verlagerung des verlagerbaren Wandabschnitts relativ zur Kopplungsformation durch die Zugangsöffnung aus der Hülle ausschiebbar ist. Oben bereits beschriebene Weiterbildungen und Ausgestaltungen der Hülle oder/und des Atemgas-Filters sind Weiterbildungen der Atemgas-Filteranordnung.

Damit der Atemgas-Filter bis zu seiner Ausgabe durch die Zugangsöffnung hindurch steril in der Hülle aufbewahrt werden kann, ist die Zugangsöffnung im Aufbewahrungszustand vor einer Kopplung mit dem Filterträger oder der Filterheizung bevorzugt durch einen abnehmbaren oder zerstörbaren Verschluss verschlossen. Der Verschluss kann durch einen die Zugangsöffnung bedeckenden und von der Zugangsöffnung entfernbaren, insbesondere abziehbaren, Materialstreifen gebildet sein. Der Verschluss kann durch einen gesondert ausgebildeten Deckel gebildet sein, dessen Deckelfläche die Zugangsöffnung im Aufbewahrungszustand bedeckt und von dessen Deckelfläche ein Kragen absteht, welcher im Aufbewahrungszustand in einen von der Hülle umgebenen Innenraum einragt und dort an Innenflächenabschnitten der Hülle anliegt.

Der Verschluss kann außerdem durch einen Materialstreifen gebildet sein, welcher durch ein Ausschieben des Atemgas-Filters aus der Hülle hinaus zerstört wird, beispielsweise zerbrochen oder zerrissen wird. Der Verschluss kann als Berst-Verschluss eine als Materialschwächung ausgebildete Sollbruchstelle aufweisen, an welcher der Verschluss bei Überschreiten einer Grenzbelastung durch den gegen den Verschluss gedrückten Atemgas-Filter seinen Materialzusammenhang verliert. Die Materialschwächung kann eine Perforation oder eine Materialdünnstelle sein, beispielsweise durch Prägen oder teilweises Einschneiden über weniger als die vollständige Materialdicke und dergleichen.

Eine Mehrzahl von Atemgas-Filteranordnungen können gestapelt in einem Spenderbehälter aufgenommen sein. Der Spenderbehälter kann eine Spenderöffnung aufweisen, durch welche hindurch jeweils eine Atemgas-Filteranordnung des Stapels entnommen werden kann. Bevorzugt ist die Spenderöffnung im Gebrauchszustand des Spenderbehälters an einem geodätisch unteren Ende ausgebildet, so dass nach Entnahme einer Atemgas-Filteranordnung durch die Spenderöffnung der im Spenderbehälter verbleibende Reststapel schwerkraftgetrieben nach unten rückt und erneut eine Atemgas-Filteranordnung entnahmebereit im Spenderbehälter vor der Öffnung zur Entnahme bereitgestellt ist.

Die vorliegende Erfindung betrifft außerdem eine Beatmungsvorrichtung zur wenigstens teilweisen künstlichen Beatmung eines lebenden Patienten, umfassend:
- eine Atemgasquelle,
- eine Beatmungsleitungsanordnung, um inspiratorisches Atemgas von der Atemgasquelle zu einer patientenseitigen proximalen Atemgas-Auslassöffnung und um exspiratorisches Atemgas von einer proximalen Atemgas-Einlassöffnung weg zu leiten,
- eine Flusssensoranordnung zur quantitativen Erfassung des inspiratorischen oder/und des exspiratorischen Atemgasflusses in der Beatmungsleitungsanordnung,
- eine Druckveränderungsvorrichtung zur Veränderung des Drucks des Atemgases in der Beatmungsleitungsanordnung sowie
- eine Steuervorrichtung zum Betrieb der Atemgasquelle oder/und der Druckveränderungsvorrichtung,
wobei die Beatmungsleitungsanordnung die erfindungsgemäße Beatmungsleitung aufweist.

Die Atemgasquelle kann ein Gebläse sein, welches aus der Umgebungsatmosphäre Umgebungsluft schöpft, kann ein Vorratsbehälter mit Atemgas sein oder kann eine Anschlussformation zur Verbindung mit einer Gebäudeinstallation zur Atemgasversorgung sein, wie sie häufig in Kliniken vorhanden ist.

Die Druckveränderungsvorrichtung kann ebenfalls das Gebläse sein, das auch Teil der Atemgasquelle ist. Zusätzlich oder alternativ kann die Druckveränderungsvorrichtung wenigstens ein Ventil umfassen.

Die vorliegende Offenbarung wird nachfolgend anhand der beiliegenden Zeichnungen näher erläutert werden. Es stellt dar:
- Fig. 1: eine schematische Explosionsdarstellung einer offenbarungsgemäßen Beatmungsvorrichtung,
- Fig. 2: eine grobschematische Explosionsansicht einer ersten Ausführungsform einer erfindungsgemäßen Beatmungsleitung als Komponente der Beatmungsvorrichtung von Figur 1,
- Fig. 3: eine grobschematische perspektivische Ansicht eines Wechsels von Atemgas-Filtern (Filtertausch) an der Beatmungsleitung von Figur 2,
- Fig. 4A: eine grobschematische Schnittansicht des Filterträgers der Figuren 2 und 3 mit darin in der Betriebsposition angeordnetem Atemgas-Filter,
- Fig. 4B: eine Vergrößerungsansicht des eingekreisten Details von Figur 4A,
- Fig. 5: eine grobschematische perspektivische Ansicht einer zweiten Ausführungsform einer erfindungsgemäßen Beatmungsleitung als Komponente der Beatmungsvorrichtung von Figur 1,
- Fig. 6: eine grobschematische perspektivische Ansicht der Beatmungsleitung von Figur 5 während eines Filtertauschs,
- Fig. 7A: eine grobschematische perspektivische Längsschnittansicht der Beatmungsleitung der Figuren 5 und 6 vor einem Filtertausch,
- Fig. 7B: eine Vergrößerungsansicht des markierten Rechteckbereichs von Figur 7A,
- Fig. 8A: eine grobschematische perspektivische Längsschnittansicht der Beatmungsleitung der Figuren 5 und 6 nach einem Filtertausch,
- Fig. 8B: eine Vergrößerungsansicht des markierten Rechteckbereichs von Figur 8A, und
- Fig. 9: eine grobschematische perspektivische Ansicht eines Spenderbehälters zur vereinzelten Ausgabe von Atemgas-Filteranordnungen der vorliegenden Anmeldung.

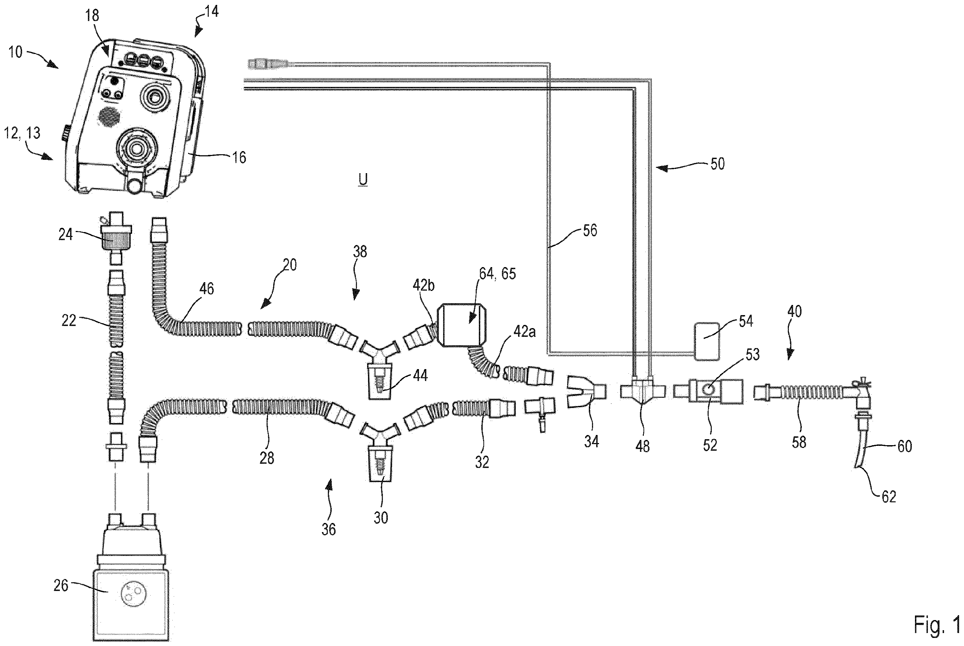

In Figur 1 ist eine offenbarungsgemäße Ausführungsform einer Beatmungsvorrichtung allgemein mit 10 bezeichnet. Die Beatmungsvorrichtung 10 umfasst eine Atemgasquelle 12 in Gestalt eines Gebläses sowie eine Steuervorrichtung 14 zur Einstellung von Betriebsparametern der Atemgasquelle 12. Die Atemgasquelle 12 und die Steuervorrichtung 14 sind im selben Gehäuse 16 aufgenommen. In diesem Gehäuse befinden sich auch an sich bekannte Ventile, wie ein Inspirationsventil und ein Exspirationsventil. Diese sind in Figur 1 jedoch nicht eigens dargestellt.

Die Steuervorrichtung 14 der Beatmungsvorrichtung 10 weist eine Eingabe/Ausgabeeinrichtung 18 auf, welche zahlreiche Schalter, wie Tastschalter und Drehschalter, umfasst, um erforderlichenfalls Daten in die Steuervorrichtung 14 eingeben zu können. Das Gebläse der Atemgasquelle 12 kann in seiner Förderleistung durch die Steuervorrichtung 14 verändert werden, um die Menge an Atemgas, das von der Atemgasquelle pro Zeiteinheit gefördert wird, zu verändern. Die Atemgasquelle 12 ist daher im vorliegenden Ausführungsbeispiel auch eine Druckveränderungsvorrichtung 13 der Beatmungsvorrichtung 10.

An die Atemgasquelle 12 ist eine Beatmungsleitungsanordnung 20 angeschlossen, welche im vorliegenden Beispiel sieben flexible Schläuche umfasst. Ein erster inspiratorischer Beatmungsschlauch 22 verläuft von einem zwischen der Atemgasquelle 12 und ihm selbst angeordneten optionalen Filter 24 zu einer Konditionierungsvorrichtung 26, wo das von der Atemgasquelle 12 gelieferte Atemgas auf einen vorgegebenen Feuchtegrad befeuchtet und gegebenenfalls mit Aerosol-Medikamenten versehen wird. Der Filter 24 filtert und reinigt die vom Gebläse als der Atemgasquelle 12 gelieferte Umgebungsluft.

Ein zweiter inspiratorischer Beatmungsschlauch 28 führt von der Konditionierungsvorrichtung 26 zu einer inspiratorischen Wasserfalle 30. Ein dritter inspiratorischer Beatmungsschlauch 32 führt von der Wasserfalle 30 zu einem Y-Verbinder 34, welcher die distale Inspirationsleitung 36 und die distale Exspirationsleitung 38 zu einer kombinierten proximalen inspiratorisch-exspiratorischen Beatmungsleitung 40 verbindet.

Vom Y-Verbinder 34 zurück zum Gehäuse 16 verläuft ein erster exspiratorischer Beatmungsschlauch 42a zu einer Atemgas-Filtervorrichtung 64. Von dieser führt ein zweiter exspiratorischer Beatmungsschlauch 42b zu einer exspiratorischen Wasserfalle 44 und von dort ein dritter exspiratorischer Beatmungsschlauch 46 zum Gehäuse 16, wo das exspiratorische Atemgas über ein nicht dargestelltes Exspirationsventil in die Umgebung U abgelassen wird.

Auf der patientennahen kombinierten inspiratorisch-exspiratorischen Seite des Y-Verbinders 34 folgt unmittelbar auf den Y-Verbinder 34 ein Durchflusssensor 48, hier: ein Differenzdruck-Durchflusssensor 48, welcher den inspiratorischen und exspiratorischen Fluss an Atemgas zum Patienten hin und vom Patienten weg erfasst. Eine Leitungsanordnung 50 überträgt den beiderseits eines Strömungshindernisses im Durchflusssensor 48 herrschenden Gasdruck an die Steuervorrichtung 14, die aus den übertragenen Gasdrücken und insbesondere aus der Differenz der Gasdrücke die Menge an pro Zeiteinheit strömendem inspiratorischem und exspiratorischem Atemgas errechnet.

In Richtung vom Y-Verbinder 34 weg folgt zum Patienten hin auf den Durchflusssensor 48 eine Messküvette 52 zur nichtdispersiven Infrarot-Erfassung eines vorbestimmten Gasanteils im Atemgas. Ermittelt werden soll im dargestellten Beispiel der Anteil an CO₂ im Atemgas. Dabei ist der CO₂-Anteil bevorzugt sowohl im inspiratorischen Atemgas wie auch im exspiratorischen Atemgas von Interesse, da die Änderung des CO₂-Anteils zwischen Inspiration und Exspiration ein Maß für die Stoffwechselfähigkeit der Patientenlunge ist.

Die Sensorbaugruppe 54 ist an die Messküvette 52 derart lösbar ankoppelbar, dass die Sensorbaugruppe 54 die Messküvette 52 durch Fenster 53 mit Infrarotlicht durchstrahlen kann. Aus der spektralen Intensität des Infrarotlichts nach Durchstrahlung der Messküvette 52 kann in an sich bekannter Weise anhand des Ausmaßes an Absorption von Infrarotlicht auf die Menge bzw. den Anteil an CO₂ im Atemgas geschlossen werden.

Die Sensorbaugruppe 54 ist über eine Datenleitung 56 mit der Steuervorrichtung 14 der Beatmungsvorrichtung 10 verbunden und überträgt über die Datenleitung 56 Intensitätsinformationen des erfassten Infrarotlichts zur Auswertung an die Steuervorrichtung 14.

Auf die Messküvette 52 folgt in Richtung zum Patienten hin ein weiteres Schlauchstück 58, an welchem ein Endotrachealtubus 60 als Beatmungsschnittstelle zum Patienten angeordnet ist. Eine proximale Öffnung 62 des Endotrachealtubus 60 ist sowohl Atemgas-Auslassöffnung, durch welche inspiratorisches Atemgas durch den Endotrachealtubus 60 in den Patienten eingeleitet wird, als auch Atemgas-Einlassöffnung, durch welche exspiratorisches Atemgas aus dem Patienten zurück in den Endotrachealtubus 60 geleitet wird.

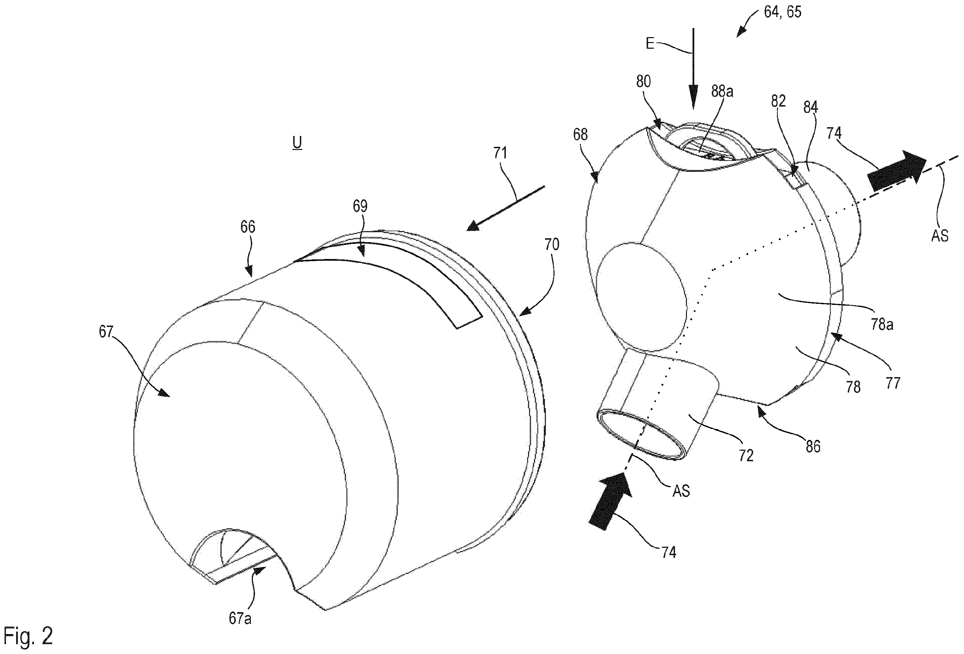

Mit Bezugszeichen 64 ist eine im ersten exspiratorischen Beatmungsschlauch 42 angeordnete beheizbare Atemgas-Filtervorrichtung bezeichnet, welche nachfolgend mit Bezugnahme auf die Figuren 2 bis 4 ausführlich beschrieben werden wird.

Die beheizbare Atemgas-Filtervorrichtung 64 bildet, wie nachfolgend mit Bezug auf Fig. 2 erläutert werden wird, eine Ausführungsform einer Beatmungsleitung 65 der vorliegenden Erfindung.

In Figur 2 ist die Atemgas-Filtervorrichtung 64 in einer grobschematischen Explosionsansicht dargestellt. Zu erkennen ist eine Filterheizung 66 und ein in die Filterheizung 66 lösbar einsetzbarer Filterträger 68.

Die Filterheizung 66 umfasst auf der vom Betrachter der Figur 2 abgewandten Seite eine Filterträgeraufnahme 70, in welche der Filterträger 68 längs des Pfeils 71 einsetzbar ist.

Das Gehäuse 67 der Filterheizung 66 weist auf ihrer Unterseite eine Aussparung 67a auf, welche der Aufnahme eines zulaufseitigen Verbindungsstutzens 72 des Filterträgers 68 dient, wenn der Filterträger 68 in seiner Betriebsposition in der Filterträgeraufnahme 70 der Filterheizung 66 aufgenommen ist.

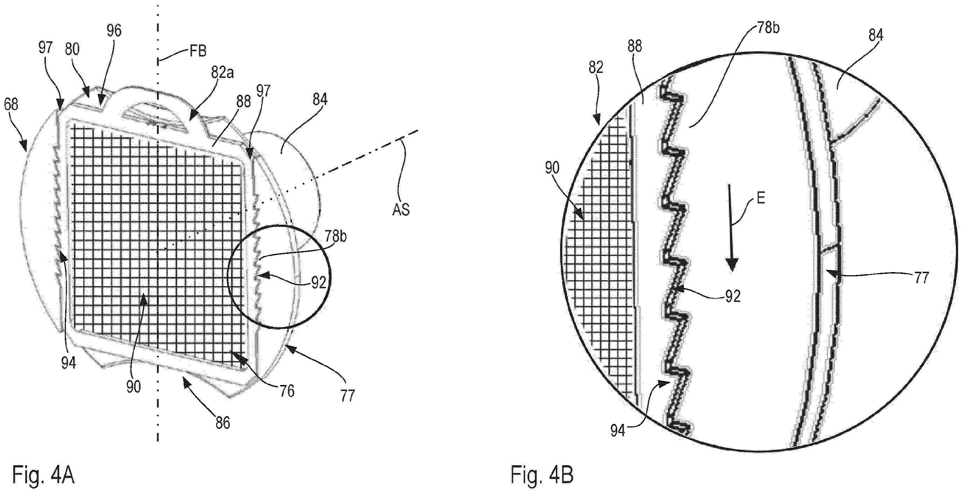

Der Verbindungsstutzen 72 bildet einen Teil der Atemgas-Strömungsführung der Beatmungsleitung 65. Im Verbindungsstutzen 72 wird durch dicke Pfeile 74 in seiner Strömungsrichtung gekennzeichnetes Atemgas, im vorliegenden Fall exspiratorisches Atemgas, in den Filterträger 68, genauer in dessen in Figur 4A erkennbaren Filteraufnahmeraum 76 im Filterträgergehäuse 77 eingeleitet. Die Verbindungsstutzen 72 können zur strömungsmechanischen Anbindung des ersten exspiratorischen Beatmungsschlauchs 42a dienen. Das Atemgas 74 strömt dabei längs eines den beispielhaft zylindrisch ausgebildeten Verbindungsstutzen 72 zentral durchsetzend gedachten virtuellen Atemgas-Strömungspfads AS, welcher im Beispiel des zylindrischen Verbindungsstutzens 72 mit dessen Zylinderachse zusammenfällt. Im Inneren des Filterträgers 68 ist der weitere, durch die Wandung 78 des Filterträgers 68 verdeckte Verlauf des virtuellen Atemgas-Strömungspfads AS punktiert angedeutet.

In bezogen auf den virtuellen Atemgas-Strömungspfad AS radialem Abstand von diesem weist die Wandung 78 des Filterträgers 68 eine Einführöffnung 80 auf, durch welche hindurch ein Atemgas-Filter 82 in den Filterträger 68 längs der Einführrichtung E eingeschoben wurde. Der Atemgas-Filter 82 in Figur 2 befindet sich in seiner Betriebsstellung, in welcher er das den Filterträger 68 durchströmende Atemgas 74 reinigt.

Die Wandung 78 des Filterträgers 68 weist im dargestellten Beispiel einen kegelstumpfförmigen Abschnitt 78a auf. Dieser Abschnitt kann jedoch eine beliebige andere Gestalt aufweisen. Der kegelstumpfförmige Abschnitt 78a bietet den Vorteil einer sicheren großflächigen Ankopplung an die Filterheizung 66, um über die Fläche des kegelstumpfförmigen Abschnitts 78a gleichmäßig eine ausreichend große Wärmemenge pro Zeiteinheit übertragen zu können.

Nach Durchgang durch den Filteraufnahmeraum 76 und den gegebenenfalls darin aufgenommenen Atemgas-Filter 82 strömt das Atemgas 74 durch den auslassseitigen Verbindungsstutzen 84 aus dem Filterträger 68 aus.

Der Filterträger 68 kann auf seiner Auslassseite wiederum einen kegelförmigen Abschnitt aufweisen, welcher sich ausgehend vom Filteraufnahmeraum 76 verjüngt, wobei der auslassseitige Verbindungsstutzen 84, welcher wiederum zur strömungsmechanischen Anbindung eines Beatmungsschlauchs, etwa des zweiten exspiratorischen Beatmungsschlauch 42b dienen kann, etwa zentral auf der Auslassseite des Filterträgers 68 angeordnet ist. Alternativ kann der auslassseitige Verbindungsstutzen 84 exzentrisch auf der Auslassseite des Filterträgers 68 angeordnet sein, wenn dies für den weiteren Leitungsverlauf vorteilhaft ist.

Wie in Figur 2 gut zu erkennen ist, sind der zulaufseitige Abschnitt des Atemgas-Strömungspfads AS im Bereich des zulaufseitigen Verbindungsstutzens 72 und der auslassseitige Abschnitt des Atemgas-Strömungspfads AS im Bereich des auslassseitiges Verbindungsstutzens 84 zueinander abgewinkelt, um trotz des vom Filterträger 68 auskragenden zulaufseitigen Verbindungsstutzens 72 eine möglichst große Fläche des Filterträgers 68 zusammenhängend von der Filterheizung 66 umgebend in Wärmeübertragungsbeziehung mit dieser anordnen zu können.

Besonders vorteilhaft weist die Wandung 67 der Filterheizung 66 eine Durchlassöffnung 69 auf, welche dann, wenn sich der Filterträger 68 in der Filterheizung 66 in seiner betriebsbereiten Stellung befindet, mit der Einführöffnung 80 des Filterträgers 68 längs der Einführrichtung E fluchtet. Dadurch ist es möglich, einen Atemgas-Filter 82 durch die Durchlassöffnung 69 und die Einführöffnung 80 in den Filterträger 68 einzuführen, ohne den Filterträger 68 hierfür aus der Filterheizung 66 entnehmen zu müssen. Die Filterheizung 66 kann vom Filterträger 68 abgenommen werden, ohne dass hierfür die Beatmungsleitung geöffnet oder/und der Atemgasstrom unterbrochen werden muss.

Die Anordnung der Einführöffnung 80 derart, dass sie in einer vom Atemgas-Strömungspfad AS radial entfernt gelegenen Wandung 78 des Filterträgers 68 ausgebildet ist, ermöglicht das Einführen eines Atemgas-Filters 82 in den Filteraufnahmeraum 76, ohne dass hierfür der Strömungspfad des Atemgases 74 und damit die künstliche Beatmung des Patienten unterbrochen werden müsste.

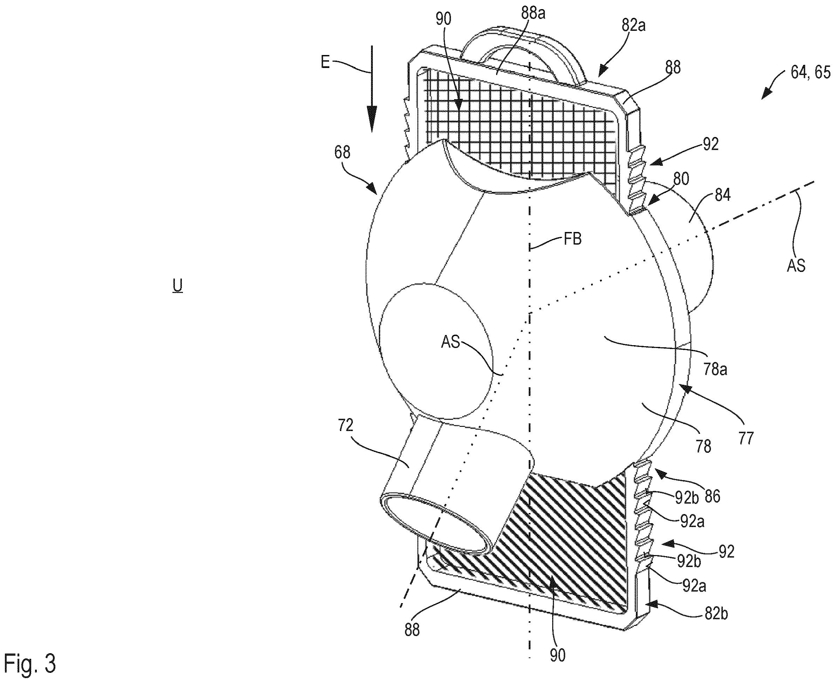

In Figur 3 ist ein besonders vorteilhafter, weil ebenso einfacher wie hygienischer Filterwechsel gezeigt. Ein verbrauchter Atemgas-Filter 82b wird durch Einführen eines neuen Atemgas-Filters 82a durch die Einführöffnung 80 in Einführrichtung E aus einer bezüglich der Einführrichtung E der Einführöffnung 80 an der Wandung 78 des Filterträgers 68 entgegengesetzt gegenüberliegenden Entnahmeöffnung 86 ausgeschoben. Der verbrauchte Atemgas-Filter 82b muss folglich zur Entnahme aus dem Filterträger 68 nicht berührt werden. Ist etwa unter der Entnahmeöffnung 86 ein Auffangbehälter angeordnet, so kann der verbrauchte Atemgas-Filter 82b durch den neuen Atemgas-Filter 82a einfach in den Auffangbehälter ausgedrückt bzw. ausgeschoben werden. Anschließend kann der verbrauchte Atemgas-Filter 82b, wiederum ohne erforderliche Berührung durch Bedienpersonal, entsorgt werden.

Die Atemgas-Filter 82 weisen einen Filterrahmen 88 auf, welche im dargestellten Beispiel ein ebenes Filtermaterial 90 umlaufend umgeben. Dadurch kann von dem in den Filterträger 68 einzuführenden neuen Atemgas-Filter 82a ausreichend Kraft auf den aus dem Filterträger 68 zu entnehmenden verbrauchten Atemgas-Filter 82b ausgeübt werden.

Die Filterrahmen 88 sind aus gasundurchlässigem Material, sodass ein in Figur 2 in der Betriebsposition des Atemgas-Filters 82 aus der Einführöffnung 80 auskragender Rahmenabschnitt 88a die Einführöffnung 80 - und ein entgegengesetzter Rahmenabschnitt ebenso die Entnahmeöffnung 86 - abdichten kann. Somit wird gleichzeitig mit der Einführung eines Atemgas-Filters 82 in dessen Betriebsposition im Filterträger 68 der den Atemgas-Filter 82 aufnehmende Filteraufnahmeraum 76 durch den Atemgas-Filter 82, genauer durch dessen Rahmen 88 gegenüber der Außenumgebung U abgedichtet. An der Entnahmeöffnung 86 kann ein Abschnitt des Rahmens 88 des in die Betriebsposition eingeführten Atemgas-Filters 82 aus der Entnahmeöffnung 86 hinausragen. Dies muss jedoch nicht so sein.

An den Rahmenseiten der Filterrahmen 88 ist eine als Verzahnung, insbesondere Sägezahn-Verzahnung mit aufeinanderfolgenden unterschiedlich geneigten Zahnflächen 92 und 92b, ausgebildete Hemmungsgegenformation 92 vorgesehen, welche mit einer besonders gut in Figur 4B erkennbaren komplementär ausgebildeten Hemmungsformation 94 des Filterträgers 68 zusammenwirkt, um nur eine Bewegung eines Atemgas-Filters 82 in Einführrichtung E durch die Einführöffnung 80 relativ zum Filterträger 68 zu gestatten und um eine Bewegung des Atemgas-Filters 82 in die entgegengesetzte Richtung zu hemmen.

Ein Atemgas-Filter 82 ist somit längs einer bevorzugten geradlinigen, knickfreien Führungsbahn FB in den Filterträger 68 bzw. in den Filteraufnahmeraum 76 einführbar und aus diesem entnehmbar. Der Atemgas-Filter 82 ist somit längs der geradlinigen, knickfreien Führungsbahn FB durch den Filterträger 68 hindurch bewegbar.

Wie in den Figuren 4A und 4B zu erkennen ist, ist an einem den Filteraufnahmeraum 76 seitlich begrenzenden Wandungsabschnitt 78b der Wandung 78 des Filterträgers 68 eine zur Hemmungsgegenformation 92 komplementäre Hemmungsformation 94 ausgebildet, welche mit der Hemmungsgegenformation 92 derart formschlüssig zusammenwirkt, dass der Atemgas-Filter 82 nur unidirektional längs der Führungsbahn FB durch den Filterträger 68 hindurch bewegbar ist. Die Hemmungsgegenformation 92 und die Hemmungsformation 94 müssen nicht streng komplementär miteinander ausgebildet sein. Es genügt, wenn die gewünschte Hemmungswirkung erreicht wird.

Die Einführöffnung 80 und die Entnahmeöffnung 86 sind Endbereiche eines Kanals 96, welcher den Filterträger 68 längs der Führungsbahn FB durchsetzt und welcher den Filteraufnahmeraum 76 bildet. Die seitlichen Begrenzungen des Kanals 96 durch den Filterträger 68 bzw. dessen Wandung 78 bildet eine Führungsstruktur 97, welche aufgrund ihrer Abmessungen eine lediglich unidirektional-translatorische, also rotationsfreie Bewegung des Atemgas-Filters 82 durch die Einführöffnung 80 in die Betriebsposition und aus der Betriebsposition durch die Entnahmeöffnung 86 ermöglicht. Somit ist der Atemgas-Filter 82 in seiner Betriebsposition vorteilhaft spielfrei und unter Ausbildung einer Gasdichtung an der Einführöffnung 80 und der Entnahmeöffnung 86 im Filterträger 68 aufgenommen.

Der Filter 24 kann abweichend von der Darstellung in Figur 1 weggelassen sein oder kann wie die Atemgas-Filtervorrichtung 64 bzw. die Beatmungsleitung 65 ausgebildet sein.

Der Anordnungsort der Atemgas-Filtervorrichtung 64 in der Beatmungsleitungsanordnung 20 im Ausführungsbeispiel ist lediglich beispielhaft. Alternativ zur Darstellung von Fig. 1 kann die Atemgas-Filtervorrichtung 64 auch zwischen der Wasserfalle 44 und dem Exspirationsventil angeordnet sein.

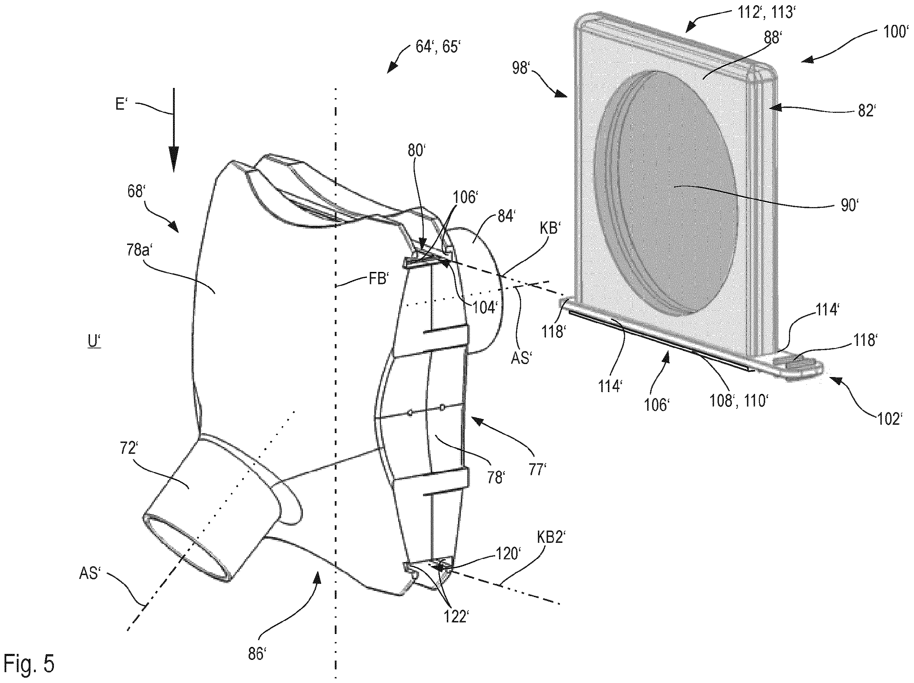

In den Figuren 5 bis 8B ist eine zweite Ausführungsform einer Beatmungsleitung 65' mit einer Atemgas-Filtervorrichtung 64' dargestellt. Gleiche und funktionsgleiche Bauteile und Bauteilabschnitte sind in der zweiten Ausführungsform mit den gleichen Bezugszeichen versehen wie in der ersten Ausführungsform der Figuren 1 bis 4B, jedoch unter Zusatz eines Apostrophen. Die zweite Ausführungsform wird nachfolgend nur in dem Umfang beschrieben als sie sich von der zuvor beschriebenen ersten Ausführungsform unterscheidet, auf deren Beschreibung ansonsten auch zur Erläuterung der zweiten Ausführungsform ausdrücklich verwiesen wird.

Die Beatmungsleitung 65' der zweiten Ausführungsform mit ihrer Atemgas-Filtervorrichtung 64' ist lediglich aus Gründen der besseren Übersichtlichkeit ohne Filterheizung dargestellt. Auch die Atemgas-Fördervorrichtung 64' der zweiten Ausführungsform kann eine Filterheizung aufweisen.

Der Atemgas-Filter 82' ist in Figur 5 in seinem Aufbewahrungszustand dargestellt, in welchem er in einer Hülle 98' aufgenommen ist. Die Hülle 98' und der darin aufgenommene Atemgas-Filter 82' bilden eine Atemgas-Filteranordnung 100'.

Die Hülle 98' und damit die Atemgas-Filteranordnung 100' insgesamt weisen auf einer Seite eine Kopplungsformation 102' auf, welche der Herstellung eines Kopplungseingriffs mit einer Kopplungsgegenformation 104' am Gehäuse 77' des Filterträgers 68' dient. Die Hülle 98' selbst ist im dargestellten Beispiel aus einem Elastomermaterial, beispielsweise aus Gummi oder aus Silikon hergestellt und umgibt den Atemgas-Filter 82' mit geringem Spaltmaß oder liegt unmittelbar am Rahmen 88' des Atemgas-Filters 82' an.

Der Atemgas-Filter 82' ist ohne Hemmungsformation dargestellt, weist jedoch bevorzugt eine solche auf.

Die Kopplungsformation 102' umgibt eine in Figur 5 nicht erkennbare Zugangsöffnung 106', durch welche hindurch der Atemgas-Filter 82' aus der Hülle 98' ausgebbar ist. Die Zugangsöffnung 106' ist durch einen Verschluss 108' in Gestalt einer auf die Kopplungsformation 102' aufgesiegelten Folie 110' verschlossen und kann geöffnet werden, indem der Atemgas-Filter 82' durch die mit einer Sollbruchstelle versehene Folie 110' hindurchgedrückt wird. Hierzu kann wenigstens ein Wandabschnitt 113' an dem der Kopplungsformation 102' entgegengesetzten Längsende 112' der elastomeren Hülle 98' verformend zur Kopplungsformation 102' hin verlagert werden.

Die Kopplungsformation 102' umfasst beiderseits der Zugangsöffnung 106' je einen über die gesamte Erstreckungslänge der Hülle 98' und darüber hinaus durchgehend verlaufenden Vorsprung 114', welcher bezüglich der Haupterstreckungsebene der Hülle 98' vorsteht. Die beiden Vorsprünge 114' sind längs der zur Führungsbahn FB' orthogonalen Kopplungsbahn KB' in jeweils eine Nut 116' der Kopplungsgegenformation 104' einschiebbar. Griffabschnitte 118' an beiden Längsenden der Kopplungsformation 102' erleichtern die Handhabung der Hülle 98' bzw. der Atemgas-Filteranordnung 100' während der Einführung der Vorsprünge 114' in die Nuten 116' und während der Verschiebung der Hülle 98' längs der Kopplungsbahn KB'.

Die beiden Nuten 116' erstrecken sich beiderseits der Einführöffnung 80' längs der Kopplungsbahn KB' über die gesamte Länge der Einführöffnung 80' und beiderseits der Einführöffnung 80' darüber hinaus.

Im Gegensatz zur ersten Ausführungsform weist der Rahmen 88' des Atemgas-Filters 82' beispielhaft eine kreisrunde Öffnung auf, durch welches hindurch das ebene Filtermaterial 90' für den Atemgasstrom zur Durchströmung zugänglich ist. Selbstverständlich kann auch die Öffnung im Rahmen 88' rechteckig oder allgemein polygonal sein.

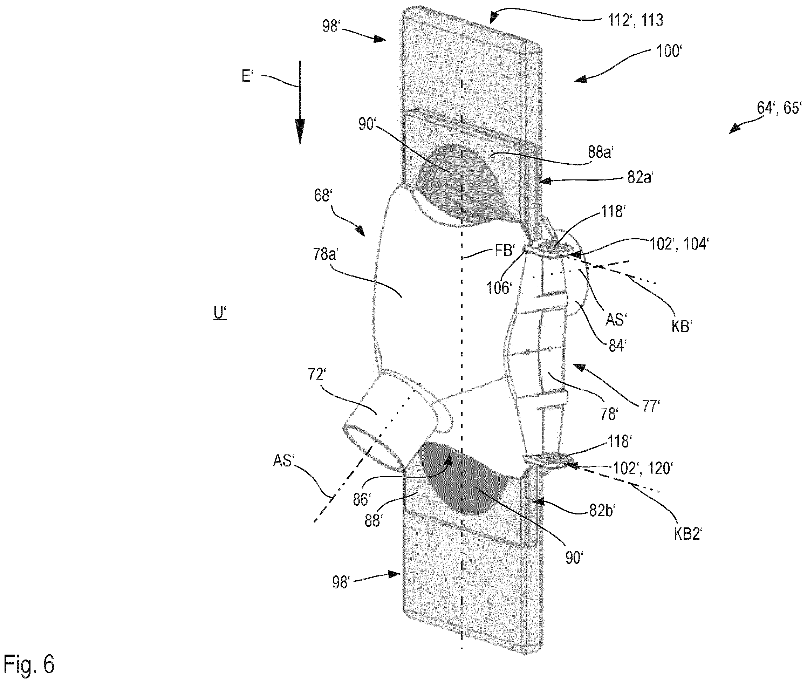

In Figur 6 ist ein Filtertausch von Atemgas-Filtern 82' der zweiten Ausführungsform gezeigt. Die Darstellung von Figur 6 entspricht daher der Darstellung von Figur 3, jedoch unter Verwendung der zweiten Ausführungsform der Atemgas-Filter 82'.

Die Atemgas-Filteranordnung 100' wurde nach Herstellung eines Kopplungseingriffs zwischen der Kopplungsformation 102' und der Kopplungsgegenformation 104', genauer zwischen den Vorsprüngen 114' und den zugeordneten Nuten 116', in den Übergabezustand verbracht, in welchem die Zugangsöffnung 106' und die Einführöffnung 80' längs der Führungsbahn FB', die vorliegend einen Einführbewegungsweg darstellt, miteinander fluchten.

In dem bezeichneten Übergabezustand wurde durch Druck auf den Wandabschnitt 113' am Längsende 112' der Hülle 98' bzw. der Atemgas-Filteranordnung 100' das Längsende 112' zur Zugangsöffnung 106' unter Verformung der Hülle 98' verlagert und dadurch der Verschluss 108' gebrochen. Durch die so geöffnete Zugangsöffnung 106' wird ein neuer Atemgas-Filter 82a' längs der Führungsbahn FB' in den Filteraufnahmeraum des Filterträgers 68' eingeführt und dabei gleichzeitig ein alter bzw. verbrauchter Atemgas-Filter 82b' längs derselben Führungsbahn FB' aus dem Filteraufnahmeraum durch die Entnahmeöffnung 86' ausgeschoben. Die Führungsbahn FB' ist daher auch ein Entnahmebewegungsweg. Ebenso ist die Einführrichtung E bzw. E' auch die Entnahmerichtung E bzw. E'.

Als ein oben genannter Auffangbehälter wurde vor Beginn des Filtertauschs eine weitere Hülle 98', welche mit der zuvor beschriebenen Hülle 98' identisch ist, am Filterträger 68' angeordnet. Hierzu weist der Filterträger 68' eine weitere Kopplungsgegenformation 120' auf, welche identisch wie die oben genannte Kopplungsgegenformation 104' ausgestaltet ist. Die weitere Kopplungsgegenformation 120' ist lediglich auf der anderen Seite des Atemgas-Strömungspfads AS' angeordnet als die Kopplungsgegenformation 104'. Das oben zur Kopplungsgegenformation 104' Gesagte gilt somit auch für die weitere Kopplungsgegenformation 120'.

Die weitere Hülle 98' wurde mit ihrer Kopplungsformation 102', genauer mit ihren Vorsprüngen 114', in Kopplungseingriff mit der weiteren Kopplungsgegenformation 120', genauer mit deren Nuten 122', gebracht. Die weitere Hülle 98' befindet sich in Figur 6 in einem Ausgabezustand, in welchem die Zugangsöffnung der weiteren Hülle 98' mit der Entnahmeöffnung 86' längs der Führungsbahn FB' fluchtet. Somit wird der verbrauchte Atemgas-Filter 82b' durch Einführen des neuen Atemgas-Filters 82a' in die weitere Hülle 98' verdrängt und kann nach erfolgtem Filtertausch, umgeben von der weiteren Hülle 98', ohne Kontakt mit einer Person vom Filterträger 68' entfernt und entsorgt werden.

Durch die Kopplungseingriffe der beiden Höhen 98' mit den Kopplungsgegenformationen 104' und 120' werden die Einführöffnung 80' und die Entnahmeöffnung 86' während des Filtertauschs gegenüber der Außenumgebung U' weitgehend abgedichtet, sodass ein in der Beatmungsleitung 65' herrschender Atemgasdruck durch den Filtertausch in vorteilhafterweise nicht beeinträchtigt wird. So kann ein PEEP auch während des Filtertauschs aufrechterhalten werden.

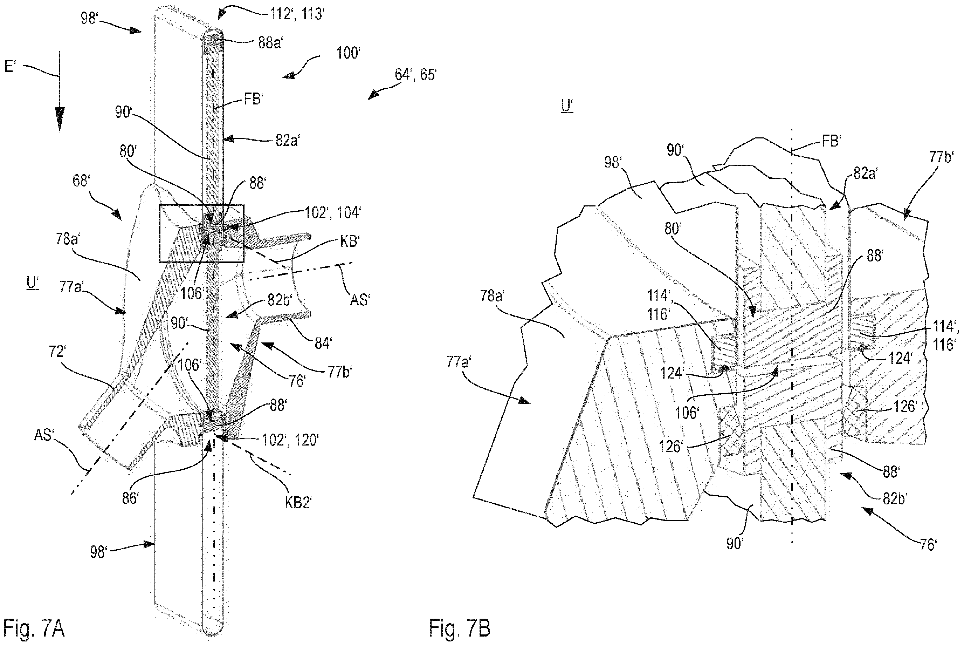

Eine besonders vorteilhafte Dichtungssituation in der Umgebung sowohl der Einführöffnung 80' als auch der Entnahmeöffnung 86' wird nachfolgend anhand der Figuren 7A bis 8B erläutert.

In Figur 7A ist eine zum Filtertausch bereite Atemgas-Filtervorrichtung 64' im Längsschnitt dargestellt. Die Atemgas-Filteranordnung 100' befindet sich im Übergabezustand, sodass der neue Atemgas-Filter 82a' bereit ist zum Filtertausch. Die Verschlussfolie 110' wurde in dem Fall der Figur 7A vor dem Herstellen des Kopplungseingriffs der Kopplungsformation 102' mit der Kopplungsgegenformation 104' entfernt. In der Betriebsposition im Filteraufnahmeraum 76' ist ein verbrauchter Atemgas-Filter 82b' angeordnet. Außerdem ist eine leere Hülle 98' mit ihrer Kopplungsformation 102' in Kopplungseingriff mit der weiteren Kopplungsgegenformation 120' und befindet sich im Ausgabezustand, sodass die leere Hülle 98' bereit ist, den gebrauchten Atemgas-Filter 82b' aufzunehmen.

In Figur 7B ist der in Figur 7A gekennzeichnete Rechteckabschnitt vergrößert dargestellt, um die Dichtungssituation an der Atemgas-Filtervorrichtung 64' vor dem Filtertausch darzustellen.

In Figur 7B ist gut zu erkennen, wie die beiderseits der Zugangsöffnung 106' an der Hülle 98' von der Zugangsöffnung 106' weg vorstehenden Vorsprünge 114' jeweils in ihre zugeordnete Nut 116' an der Kopplungsgegenformation 104' des Filterträgergehäuses 77' eingreifen. Der Eingriff erstreckt sich über die gesamte Länge der Nut 116'.

An der entgegen der Einführrichtung E' weisenden Fläche einer jeden Nut 116' ist jeweils eine Dichtungsstruktur in Gestalt eines Dichtungsstreifens 124' angeordnet, welcher längs der Kopplungsbahn KB' verläuft und von der Fläche der Nut 116` in den von der Nut 116` umschlossenen Nutraum hinein vorsteht. Jeder Dichtungsstreifen 124' liegt daher mit seiner von der ihn tragenden Nutfläche fernliegenden Endseite an einer der Nutfläche zugewandten Fläche des in die Nut 116' eingeführten Vorsprungs 114' an. Der Dichtungsstreifen 124' dichtet somit einen zwischen einem jeden Vorsprung 114' und der zugeordneten Nut 116', in die er eingreift, bestehenden Spalt ab. Es besteht somit während eines Filtertauschs durch die Nut 116` kein direkter Strömungsweg zwischen dem Filteraufnahmeraum 76', in welchem ein gewünschter Atemgasdruck herrscht, und der Umgebung U'.

Abweichend von der Darstellung können die Dichtungsstreifen zusätzlich oder alternativ an jeder anderen Wand der Nut 116' angeordnet sein, welcher im Übergabezustand ein Außenwandabschnitt eines Vorsprungs 114' gegenüberliegt.

Der Dichtungsstreifen 124' kann beispielsweise eine längs der Kopplungsbahn KB' auf die betreffende Nutfläche aufgetragene Silikonspur sein oder kann ein beliebiges anderes weichelastisches Dichtungsmaterial sein.

Zusätzlich zu der Dichtungsstruktur in Gestalt der Dichtungsstreifen 124' ist an den beiden Filterträger-Gehäusebauteilen 77a' und 77b' des Filterträgergehäuses 77' jeweils eine Dichtungsformation 126' angeordnet, welche vorzugsweise geschlossen um die Atemgas-Strömungsbahn AS' umläuft.

Solange ein Atemgas-Filter 82' sich korrekt in der Betriebsposition angeordnet im Filteraufnahmeraum 76' befindet, ist der vom Atemgasstrom durch das Filterträgergehäuse 77' hindurch erreichbare Teil des Filtermaterials 90' durch die beiderseits des Filtermaterials 90' am Rahmen 88' des Atemgas-Filters 82' anliegenden Dichtungsformationen 126' von der Außenumgebung U' abgeschirmt. Die Dichtungsformationen 126' sind beiderseits des Atemgas-Filters 82' identisch ausgebildet, beispielsweise durch einen geschlossen umlaufenden Elastomerstreifen.

Dann, wenn der verbrauchte Atemgas-Filter 82b' durch den neuen Atemgas-Filter 82a' längs der Führungsbahn FB' durch die Entnahmeöffnung 86' hindurch verdrängt wird, kommt in einem Zeitabschnitt der Entnahmebewegung des verbrauchten Atemgas-Filters 82b' der Rahmen 88' abschnittsweise außer Anlageeingriff mit den Dichtungsformationen 126', weil das bestimmungsgemäß vom Atemgasstrom erreichbare Filtermaterial 90' mit den Dichtungsformationen 126' überlappt. In diesem Fall können die Dichtungsformationen 126' in dem Bereich, in dem sie dem vom Atemgasstrom erreichbaren freiliegenden Teil des Filtermaterials 90' gegenüberliegen, die Atemgas-Strömung nicht mehr vollständig gegen die Außenumgebung abdichten. Dies geschieht dann alleine durch die Dichtungsstreifen 124', welche aufgrund ihrer Dichtungswirkung gegen die Kopplungsformation 102' weiterhin für eine Aufrechterhaltung des in der Beatmungsleitung 65' herrschenden Atemgasdrucks sorgen.

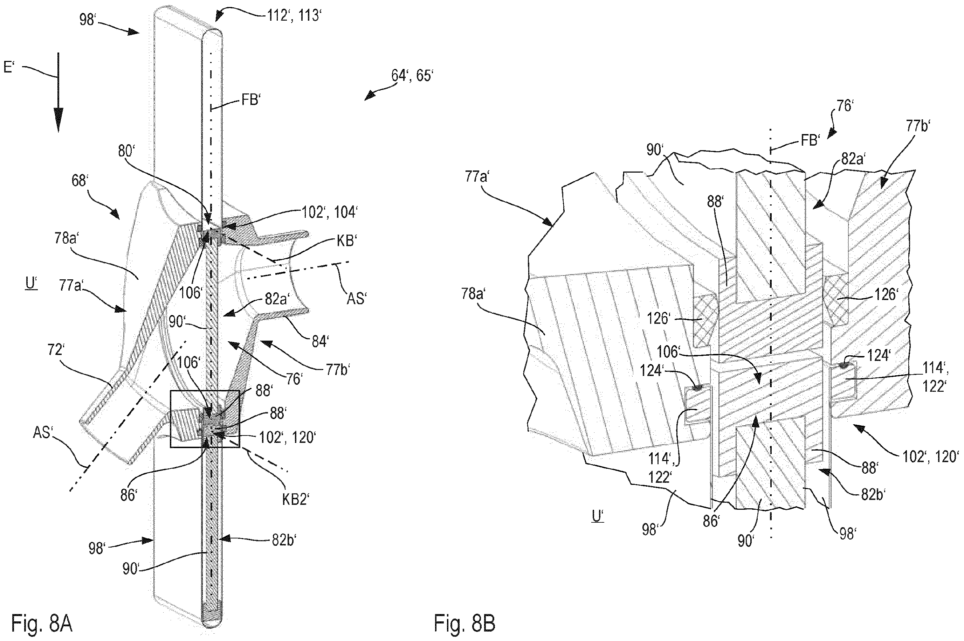

Die Darstellung der Figur 8A entspricht der Darstellung von Figur 7A, jedoch zu einem Zeitpunkt nach dem Filtertausch. Figur 8B zeigt wiederum den in Figur 8A markierten Rechteckbereich in Vergrößerung.

Der neue Atemgas-Filter 82a' wurde durch verformende Verlagerung des Wandabschnitts 113' am Längsende 112' der in den Figuren 7A und 8A oberen Hülle 98' zur Zugangsöffnung 106' hin ohne direkte Berührung durch die bedienende Person in den Filteraufnahmeraum 76' verbracht. Mit der Einführbewegung des neuen Atemgas-Filters 82a' wurde durch den Anlageeingriff der beiden Rahmen 88' des neuen Atemgas-Filters 82a' und des verbrauchten Atemgas-Filters 82b' der verbrauchte Atemgas-Filter 82b' in die in den Figuren 7A und 8A untere Hülle 98' ausgeschoben.

Der verbrauchte Atemgas-Filter 82b' kann mit der unteren Hülle 98' ohne direkten Kontakt mit dem Atemgas-Filter 82b' längs der unteren Kopplungsbahn KB2' relativ zum Filterträger 68' verschoben und somit vom Filterträger 68' gelöst werden.

Die weitere Kopplungsgegenformation 120' ist bezüglich einer zu den beiden Kopplungsbahnen KB' und KB2' parallelen Spiegelsymmetrieebene spiegelsymmetrisch. Daher weisen auch die jeweiligen Nuten 122' der weiteren Kopplungsgegenformation 120' begrenzende Flächen, welche in Entnahmerichtung bzw. in Einführrichtung E' weisen, je einen Dichtungsstreifen 124' auf (siehe Figur 8A). Es gilt wegen der genannten Spiegelsymmetrie die Beschreibung der Nuten 116' unter Beachtung der Spiegelsymmetrie auch für die Nuten 122'.

Die Hüllen 98' können von beiden Seiten des Filterträgers 68' entlang der jeweiligen Kopplungsbahn KB' und KB2' in die Kopplungsgegenformation 104' und in die weitere Kopplungsgegenformation 120' eingeführt werden.

Dann also, wenn in beiden Kopplungsgegenformationen 104' und 120' je eine Hülle 98' angeordnet ist, dichten Dichtungsstreifen 124' den Filteraufnahmeraum 76' vollständig oder wenigstens nahezu vollständig gegen die Außenumgebung U' ab.

In Figur 8A ist der näher bei der Entnahmeöffnung 86' gelegene Abschnitt der umlaufenden Dichtungsformation 126' erkennbar, die nun an dem Rahmen 88' des soeben eingeführten neuen Atemgas-Filters 82a' dichtend anliegen.

Mit der Atemgas-Filtervorrichtung 64' der zweiten Ausführungsform, wie sie in den Figuren 5 bis 8B dargestellt ist, kann ein Tausch von Atemgas-Filtern 82' bei laufendem Beatmungsbetrieb ohne Beeinträchtigung des in der Beatmungsleitung 65' herrschenden Atemgasdrucks ausgeführt werden.

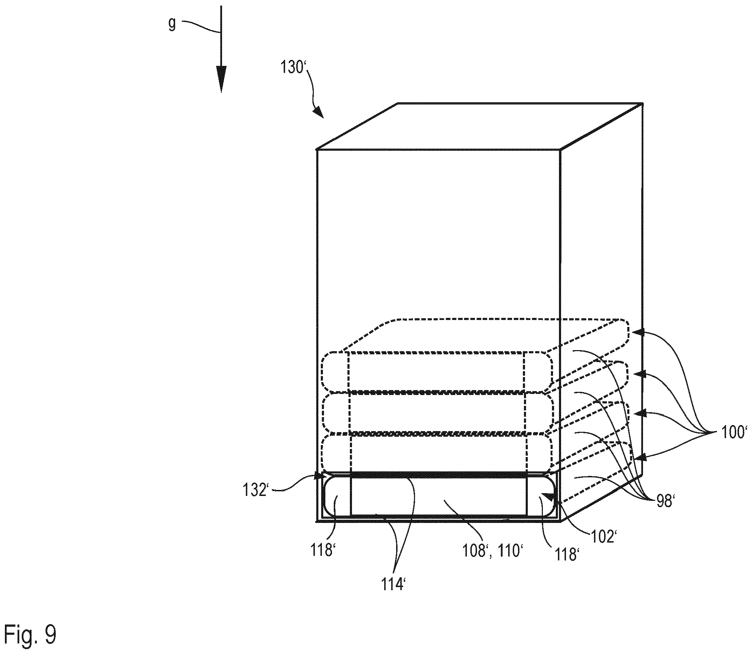

In Figur 9 ist ein vorteilhafter Spenderbehälter 130' gezeigt, welcher eine Mehrzahl von Atemgas-Filteranordnung in 100' längs der Schwerkraftwirkungsrichtung g gestapelt aufweist.

Durch eine Spenderöffnung 132' am geodätisch, also in Schwerkraftwirkungsrichtung g unteren Ende kann jeweils die unterste Atemgas-Filteranordnung 100' aus dem Spenderbehälter 130' herausgezogen werden, wobei übrigen noch im Spenderbehälter 130' verbleibenden Atemgas-Filteranordnung in 100' schwerkraftgetrieben nachrücken, sodass nach der Entnahme einer Atemgas-Filteranordnung 100' die zuvor unmittelbar über dieser liegende Atemgas-Filteranordnung 100' nun vor der Spenderöffnung 132' liegt und zur Entnahme aus dem Spenderbehälter 130' bereitgestellt ist.

Die Breite der Spenderöffnung 132' in Figur 9 längs der Schwerkraftwirkungsrichtung g ist etwas größer als die in derselben Richtung zu messende Breite der Kopplungsformation 102', sodass die Atemgas-Filteranordnung in 100' nur durch die Spenderöffnung 132' vereinzelt aus dem Spenderbehälter 130' entnehmbar sind. Bei der Entnahme der Vorderspenderöffnung 132' bereitstehenden untersten Atemgas-Filteranordnung 100' wird die unmittelbar darüber liegende Atemgas-Filteranordnung 100' an dem über der Spenderöffnung 132' gelegenen Wandabschnitt des Spenderbehälter 100' abgestreift.

## Patentansprüche

1. Beatmungsleitung (65; 65'), welche zur Führung von inspiratorischem oder/und exspiratorischem Atemgas (74) während einer wenigstens teilweisen künstlichen Beatmung eines Patienten ausgebildet ist, wobei die Beatmungsleitung (65; 65') einen sie zentral durchsetzend gedachten virtuellen Atemgas-Strömungspfad (AS; AS') definiert, längs welchem die Beatmungsleitung (65; 65') das Atemgas (74) führt, wobei die Beatmungsleitung (65; 65') einen Filterträger (68; 68') aufweist, in welchem ein vom Atemgas-Strömungspfad (AS; AS') durchsetzter Filteraufnahmeraum (76; 76') ausgebildet ist, in dem ein vom Atemgas (74) durchströmbarer Atemgas-Filter (82; 82') austauschbar aufnehmbar ist,
wobei der Filterträger (68; 68') eine den Filteraufnahmeraum (76; 76') mit radialem Abstand vom virtuellen Atemgas-Strömungspfad (AS; AS') umgebende Wandung (78; 78') mit einer mit radialem Abstand vom virtuellen Atemgas-Strömungspfad (AS) angeordneten Einführöffnung (80; 80') aufweist, durch welche hindurch der Atemgas-Filter (82; 82') in den Filteraufnahmeraum (76; 76') einführbar und in seine betriebsbereite Betriebsposition bringbar ist,
**dadurch gekennzeichnet, dass** die Wandung (78; 78') des Filterträgers (68; 68') eine mit radialem Abstand vom virtuellen Atemgas-Strömungspfad (AS; AS') angeordnete, von der Einführöffnung (80; 80') verschiedene Entnahmeöffnung (86; 86') aufweist, durch welche hindurch ein im Filteraufnahmeraum (76; 76') angeordneter Atemgas-Filter (82; 82') aus dem Filteraufnahmeraum (76; 76') entnehmbar ist.

2. Beatmungsleitung (65; 65') nach Anspruch 1,
**dadurch gekennzeichnet, dass** der Filterträger (68; 68') eine Führungsstruktur (97) aufweist, welche eine Einführbewegung des Atemgas-Filters (82; 82') von der Einführöffnung (80; 80') in den Filteraufnahmeraum (76; 76') hinein, insbesondere in die Betriebsposition, führt.

3. Beatmungsleitung (65; 65') nach Anspruch 2, **dadurch gekennzeichnet, dass** die Führungsstruktur (97) zwischen der Einführöffnung (80; 80') und der Entnahmeöffnung (86; 86') angeordnet ist, sodass sie auch eine Entnahmebewegung eines Atemgas-Filters (82; 82') aus dem Filteraufnahmeraum (76; 76') führt.

4. Beatmungsleitung (65; 65') nach einem der vorhergehenden Ansprüche,
**dadurch gekennzeichnet, dass** der Filterträger (68; 68'), insbesondere die Führungsstruktur (97), eine Hemmungsformation (94) aufweist, welche eine Bewegung des Atemgas-Filters (82; 82') durch die Einführöffnung (80; 80') in den Filteraufnahmeraum (76; 76') hinein zulässt und eine entgegengesetzt gerichtete Bewegung des Atemgas-Filters (82; 82') durch die Einführöffnung (80; 80') hemmt.

5. Beatmungsleitung (65; 65') nach einem der vorhergehenden Ansprüche,
**dadurch gekennzeichnet, dass** der Atemgas-Filter (82; 82') eine gasundurchlässige Randleiste, vorzugsweise einen ein Filtermaterial (90; 90') umgebenden gasundurchlässigen Rahmen (88; 88') aufweist, welche dann, wenn der Atemgas-Filter (82; 82') betriebsbereit im Filteraufnahmeraum (76; 76') aufgenommen ist, eine Strömungsbarriere gegen eine Durchströmung der Einführöffnung (80; 80') mit Atemgas (74) bildet.

6. Beatmungsleitung (65; 65') nach einem der vorhergehenden Ansprüche,
**dadurch gekennzeichnet, dass** die Wandung (78; 78') Teil eines den Filteraufnahmeraum (76; 76') im betriebsbereiten Zustand umgebenden Gehäuses (77; 77') des Filterträgers (68; 68') ist, wobei das Gehäuse (77; 77') als teilbares Gehäuse (77; 77') wenigstens zwei voneinander entfernbare und aneinander annäherbare Gehäuseteile (77a', 77b') aufweist.

7. Beatmungsleitung (65; 65') nach einem der vorhergehenden Ansprüche,
**dadurch gekennzeichnet, dass** der Filterträger (68; 68') beiderseits des Filteraufnahmeraums (76; 76') strömungsführende Leitungsabschnitte (72, 84; 72', 84') aufweist, deren jeweils zugeordnete Abschnitte des virtuellen Atemgas-Strömungspfads (AS; AS') miteinander einen Winkel einschließen.

8. Beatmungsleitung (65) nach einem der vorhergehenden Ansprüche,
**dadurch gekennzeichnet, dass** die Beatmungsleitung (65) eine Filterheizung (66) aufweist, welche den Filterträger (68) im betriebsbereiten Zustand wenigstens abschnittsweise umgibt.

9. Beatmungsleitung (65') nach einem der vorhergehenden Ansprüche,
**dadurch gekennzeichnet, dass** der Atemgas-Filter (82') längs eines Einführbewegungswegs (FB') in den Filteraufnahmeraum (76') einführbar ist, wobei der Atemgas-Filter (82') in einem Ausgangszustand vor seiner Einführung in den Filteraufnahmeraum (76') in einer Hülle (98') aufgenommen ist, wobei die Hülle (98') eine Kopplungsformation (102') aufweist, welche mit einer Kopplungsgegenformation (104') des Filterträgers (68') oder der Filterheizung (66) derart koppelbar ist, dass in einem Übergabezustand, in welchem eine zur Ausgabe des Atemgas-Filters (82') aus der Hülle (98') ausgebildete Zugangsöffnung (106') der Hülle (98') mit der Einführöffnung (80') längs des Einführbewegungswegs (FB') fluchtet, die Hülle (98') an der Kopplungsgegenformation (104') gehalten ist, wobei wenigstens ein Wandabschnitt (113') der Hülle (98') relativ zur Kopplungsformation (102') derart verlagerbar ist, dass der Atemgas-Filter (82') durch Verlagerung des verlagerbaren Wandabschnitts (113') relativ zur Kopplungsformation (102') durch die Zugangsöffnung (106') aus der Hülle (98') ausschiebbar ist.

10. Beatmungsleitung (65') nach Anspruch 9,
**dadurch gekennzeichnet, dass** die Kopplungsformation (102') und die Kopplungsgegenformation (104') eine Verlagerung der Hülle (98') relativ zum Filterträger (68') längs einer Kopplungsbahn (KB', KB2') ermöglicht, welche quer zum Einführbewegungsweg (FB') des Atemgas-Filters (82') in den Filterträger (68') hinein verläuft.

11. Beatmungsleitung (65') nach einem der Ansprüche 9 oder 10,
**dadurch gekennzeichnet, dass** wenigstens eine Formation (104') aus Kopplungsformation (102') und Kopplungsgegenformation (104') eine Dichtungsstruktur (124') aufweist, welche wenigstens im Übergabezustand dichtend an der jeweils anderen Formation (102') anliegt.

12. Beatmungsleitung (65') nach einem der Ansprüche 9 bis 11,
**dadurch gekennzeichnet, dass** der Atemgas-Filter (82') längs eines Entnahmebewegungswegs (FB') aus dem Filteraufnahmeraum (76') entnehmbar ist, wobei der Filterträger (68') oder die Filterheizung (66) eine weitere Kopplungsgegenformation (120') aufweist, wobei eine Hülle (98') derart mit der weiteren Kopplungsgegenformation (120') koppelbar ist, dass in einem Ausgabezustand, in welchem die Zugangsöffnung (106') der Hülle (98') mit der Entnahmeöffnung (86') längs des Entnahmebewegungswegs (FB') fluchtet, die Hülle (98') an der weiteren Kopplungsgegenformation (120') gehalten ist.

13. Beatmungsleitung (65') nach einem der vorhergehenden Ansprüche,
**dadurch gekennzeichnet, dass** der Atemgas-Filter (82') einen ein Filtermaterial (90') umgebenden gasundurchlässigen Rahmen (88') aufweist, wobei mit Abstand von der Atemgas-Strömungsbahn (AS'), vorzugsweise am Randbereich der Einführöffnung (80') oder/und der Entnahmeöffnung (86'), besonders bevorzugt um die Atemgas-Strömungsbahn (AS') geschlossen umlaufend, eine Dichtungsformation (126'), insbesondere eine Dichtlippe, vorgesehen ist, welche in der Betriebsposition des Atemgas-Filters (82') am Rahmen (88') des Atemgas-Filters (82') dichtend anliegt.

14. Beatmungsleitung (65') nach einem der Ansprüche 1 bis 13,
**dadurch gekennzeichnet, dass** sie eine Atemgas-Filteranordnung (100') aufweist, umfassend einen in einer Hülle (98') aufgenommenen Atemgas-Filter (82'), wobei die Hülle (98') eine Kopplungsformation (102') zur Kopplung mit einer Kopplungsgegenformation (104') eines Filterträgers (68') oder einer Filterheizung (66) sowie eine Zugangsöffnung (106') zur Ausgabe des Atemgas-Filters (82; 82') aus der Hülle aufweist, wobei wenigstens ein Wandabschnitt (113') der Hülle (98') relativ zur Kopplungsformation (102') derart verlagerbar ist, dass der Atemgas-Filter (82') durch Verlagerung des verlagerbaren Wandabschnitts (113') relativ zur Kopplungsformation (102') durch die Zugangsöffnung (106') aus der Hülle (98') ausschiebbar ist, wobei die Zugangsöffnung (106') im Aufbewahrungszustand vor einer Kopplung mit dem Filterträger (68') oder der Filterheizung (66) durch einen abnehmbaren oder zerstörbaren Verschluss (108') verschlossen ist.

15. Beatmungsvorrichtung (10) zur wenigstens teilweisen künstlichen Beatmung eines lebenden Patienten, umfassend:
- eine Atemgasquelle (12),
- eine Beatmungsleitungsanordnung (20), um inspiratorisches Atemgas von der Atemgasquelle (12) zu einer patientenseitigen proximalen Atemgas-Auslassöffnung (62) und um exspiratorisches Atemgas von einer proximalen Atemgas-Einlassöffnung (62) weg zu leiten,
- eine Flusssensoranordnung zur quantitativen Erfassung des inspiratorischen oder/und des exspiratorischen Atemgasflusses in der Beatmungsleitungsanordnung (20)
- eine Druckveränderungsvorrichtung (13) zur Veränderung des Drucks des Atemgases in der Beatmungsleitungsanordnung (20) sowie
- eine Steuervorrichtung (14) zum Betrieb der Atemgasquelle (12) oder/und der Druckveränderungsvorrichtung (13),
wobei die Beatmungsleitungsanordnung (20) eine Beatmungsleitung (65; 65') nach einem der vorhergehenden Ansprüche aufweist.

## Claims

1. A respiratory line (65; 65') which is designed for conveying inspiratory or/and expiratory breathing gas (74) during at least partial artificial respiration of a patient, wherein the respiratory line (65; 65') defines an imaginary virtual breathing gas flow path (AS; AS') which is conceived as extending centrally through it, along which flow path the respiratory line (65; 65') conveys the breathing gas (74), wherein the respiratory line (65; 65') has a filter carrier (68; 68') in which a filter receiving space (76; 76') which is passed through by the breathing gas flow path (AS; AS') is formed, in which a breathing gas filter (82; 82'), through which the breathing gas (74) can flow, can be replaceably accommodated,
wherein the filter carrier (68; 68') has a wall (78; 78') surrounding the filter receiving space (76; 76') with radial spacing from the virtual breathing gas flow path (AS; AS') with an introduction opening (80; 80') arranged with radial spacing from the virtual breathing gas flow path (AS), through which the breathing gas filter (82; 82') can be introduced into the filter receiving space (76; 76') and be brought into its operational operating position,
**characterized in that** the wall (78; 78') of the filter carrier (68; 68') has a removal opening (86; 86') different from the introduction opening (80; 80') and arranged with radial spacing from the virtual breathing gas flow path (AS; AS'), through which removal opening a breathing gas filter (82; 82') arranged in the filter receiving space (76; 76') can be removed from the filter receiving space (76; 76').

2. The respiratory line (65; 65') according to claim 1,
**characterized in that** the filter carrier (68; 68') has a guiding structure (97) which guides an introduction movement of the breathing gas filter (82; 82') from the introduction opening (80; 80') into the filter receiving space (76; 76'), in particular into the operating position.

3. The respiratory line (65; 65') according to claim 2,
**characterized in that** the guiding structure (97) is arranged between the introduction opening (80; 80') and the removal opening (86; 86'), so that it also guides a removal movement of a breathing gas filter (82; 82') from the filter receiving space (76; 76').

4. The respiratory line (65; 65') according to any one of the preceding claims,
**characterized in that** the filter carrier (68; 68'), in particular the guiding structure (97), has an inhibiting formation (94) which allows a movement of the breathing gas filter (82; 82') through the introduction opening (80; 80') into the filter receiving space (76; 76') and which inhibits an oppositely directed movement of the breathing gas filter (82; 82') through the introduction opening (80; 80').

5. The respiratory line (65; 65') according to any one of the preceding claims,
**characterized in that** the breathing gas filter (82; 82') has a gas impermeable shoulder, preferably a gas impermeable frame (88; 88') surrounding a filter material (90; 90'), which, when the breathing gas filter (82; 82') is accommodated in operational state in the filter receiving space (76; 76'), forms a flow barrier against throughflow of breathing gas (74) through the introduction opening (80; 80').

6. The respiratory line (65; 65') according to any one of the preceding claims,
**characterized in that** the wall (78; 78') is part of a housing (77; 77') of the filter carrier (68; 68'), which surrounds the filter receiving space (76; 76') in the operational state, wherein the housing (77; 77'), as dividable housing (77; 77'), has at least two housing portions (77a', 77b') which can be moved apart and closer together.

7. The respiratory line (65; 65') according to any one of the preceding claims,
**characterized in that** the filter carrier (68; 68'), on both sides of the filter receiving space (76; 76'), has flow-conveying line sections (72, 84; 72', 84'), of which respective associated sections of the virtual breathing gas flow path (AS; AS') enclose an angle with one another.

8. The respiratory line (65) according to any one of the preceding claims,
**characterized in that** the respiratory line (65) has a filter heater (66) which surrounds the filter carrier (68) in the operational state at least in sections.

9. The respiratory line (65') according to any one of the preceding claims,
**characterized in that** the breathing gas filter (82') can be introduced along an introduction movement path (FB') into the filter receiving space (76'), wherein the breathing gas filter (82'), in a starting state before its introduction into the filter receiving space (76'), is accommodated in a sleeve (98'), wherein the sleeve (98') has a coupling formation (102') which can be coupled to a coupling counter-formation (104') of the filter carrier (68') or of the filter heater (66) in such a manner that, in a transfer state in which an access opening (106') of the sleeve (98'), which is designed for the delivery of the breathing gas filter (82') out of the sleeve (98'), is aligned with the introduction opening (80') along the introduction movement path (FB'), the sleeve (98') is held on the coupling counter-formation (104'), wherein at least one wall section (113') of the sleeve (98') can be shifted relative to the coupling formation (102') in such a manner that the breathing gas filter (82'), as a result of displacement of the displaceable wall section (113') relative to the coupling formation (102'), can be shifted through the access opening (106') out of the sleeve (98').

10. The respiratory line (65') according to claim 9,
**characterized in that** the coupling formation (102') and the coupling counter-formation (104') enable a displacement of the sleeve (98') relative to the filter carrier (68') along a coupling path (KB', KB2') which runs transversely to the introduction movement path (FB') of the breathing gas filter (82') into the filter carrier (68').

11. The respiratory line (65') according to any one of claims 9 or 10,
**characterized in that** at least one formation (104') out of coupling formation (102') and coupling counter-formation (104') has a sealing structure (124') which, at least in the transfer state, sealingly lies against the respective other formation (102').

12. The respiratory line (65') according to any one of claims 9 to 11,
**characterized in that** the breathing gas filter (82') can be removed along a removal movement path (FB') from the filter receiving space (76'), wherein the filter carrier (68') or the filter heater (66) has an additional coupling counter-formation (120'), wherein a sleeve (98') can be coupled to the additional coupling counter-formation (120') in such a manner that, in a delivery state, in which the access opening (106') of the sleeve (98') is aligned with the removal opening (86') along the removal movement path (FB'), the sleeve (98') is held on the additional coupling counter-formation (120').

13. The respiratory line (65') according to any one of the preceding claims,
**characterized in that** the breathing gas filter (82') has a gas impermeable frame (88') surrounding a filter material (90'), wherein, with a spacing from the breathing gas flow path (AS'), preferably on the edge region of the introduction opening (80') or/and of the removal opening (86'), particularly preferably running in a closed path around the breathing gas flow path (AS'), a sealing formation (126'), in particular a sealing lip, is provided, which, in the operating position of the breathing gas filter (82'), sealingly lies against the frame (88') of the breathing gas filter (82').

14. A respiratory line (65') according to anyone of the claims 1 to 13,
**characterized in that** it comprises a breathing gas filter arrangement (100'), including a breathing gas filter (82') accommodated in a sleeve (98'), wherein the sleeve (98') has a coupling formation (102') for coupling to a coupling counter-formation (104') of a filter carrier (68') or of a filter heater (66) as well as an access opening (106') for the delivery of the breathing gas filter (82; 82') from the sleeve, wherein at least one wall section (113') of the sleeve (98') can be displaced relative to the coupling formation (102') in such a manner that the breathing gas filter (82'), as a result of displacement of the displaceable wall section (113') relative to the coupling formation (102'), can be shifted through the access opening (106') out of the sleeve (98'),
wherein the access opening (106'), in the storage state before coupling to the filter carrier (68') or to the filter heater (66), is closed by means of a removable or destructible closure (108').

15. A respiratory device (10) for at least partial artificial respiration of a living patient, including:
- a breathing gas source (12),
- a respiratory line arrangement (20), in order to convey inspiratory breathing gas from the breathing gas source (12) to a patient-side proximal breathing gas outlet opening (62) and in order to convey expiratory breathing gas away from a proximal breathing gas inlet opening (62),
- a flow sensor arrangement for quantitative acquisition of the inspiratory or/and of the expiratory breathing gas in the respiratory line arrangement (20),
- a pressure change device (13) for changing the pressure of the breathing gas in the respiratory line arrangement (20) as well as
- a control device (14) for operating the breathing gas source (12) or/and the pressure change device (13),
wherein the respiratory line arrangement (20) has a respiratory line (65; 65') according to any one of the preceding claims.

## Revendications

1. Conduit de respiration (65 ; 65') conçu pour le guidage de gaz de respiration inspiratoire et/ou expiratoire (74) pendant une ventilation artificielle au moins partielle d'un patient, dans lequel le conduit de respiration (65 ; 65') définit un chemin d'écoulement de gaz de respiration virtuel (AS ; AS') qui est prévu pour le traverser, le long duquel le conduit de respiration (65 ; 65') conduit le gaz de respiration (74), dans lequel le conduit de respiration (65 ; 65') présente un support de filtre (68 ; 68') dans lequel un espace de réception de filtre (76 ; 76') traversé par le chemin d'écoulement de gaz de respiration (AS ; AS') est configuré, dans lequel un filtre de gaz de respiration (82 ; 82') pouvant être traversé par le gaz de respiration (74) peut être logé de manière interchangeable,
dans lequel le support de filtre (68 ; 68') présente une paroi (78 ; 78') entourant l'espace de réception de filtre (76 ; 76') à une distance radiale du chemin d'écoulement de gaz de respiration virtuel (AS), avec une ouverture d'introduction (80 ; 80') disposée à une distance radiale du chemin d'écoulement de gaz de respiration virtuel (AS), à travers laquelle le filtre de gaz de respiration (82 ; 82') peut être introduit dans l'espace de réception de filtre (76 ; 76') et être amené dans sa position opérationnelle,
**caractérisé en ce que** la paroi (78 ; 78') du support de filtre (68 ; 68') présente une ouverture de prélèvement (86, 86') disposée à une distance radiale du chemin d'écoulement de gaz de respiration virtuel (AS ; AS') et différente de l'ouverture d'introduction (80 ; 80'), à travers laquelle un filtre de gaz de respiration (82 ; 82') disposé dans l'espace de réception de filtre (76 ; 76') peut être retiré de l'espace de réception de filtre (76 ; 76').

2. Conduit de respiration (65 ; 65') selon la revendication 1,
**caractérisé en ce que** le support de filtre (68 ; 68') présente une structure de guidage (97) qui guide un mouvement d'introduction du filtre de gaz de respiration (82 ; 82') depuis l'ouverture d'introduction (80 ; 80') dans l'espace de réception de filtre (76 ; 76'), en particulier dans la position opérationnelle.

3. Conduit de respiration (65 ; 65') selon la revendication 2,
**caractérisé en ce que** la structure de guidage (97) est disposée entre l'ouverture d'introduction (80 ; 80') et l'ouverture de prélèvement (86, 86'), de sorte qu'elle guide également un mouvement de prélèvement d'un filtre de gaz de respiration (82, 82') hors de l'espace de réception de filtre (76 ; 76').

4. Conduit de respiration (65 ; 65') selon l'une des revendications précédentes,
**caractérisé en ce que** le support de filtre (68 ; 68'), notamment la structure de guidage (97), présente une formation d'inhibition (94) qui autorise un mouvement du filtre de gaz de respiration (82, 82') à travers l'ouverture d'introduction (80 ; 80') dans l'espace de réception de filtre (76 ; 76') et inhibe un mouvement de sens opposé du filtre de gaz de respiration (82, 82') à travers l'ouverture d'introduction (80 ; 80').

5. Conduit de respiration (65 ; 65') selon l'une des revendications précédentes,
**caractérisé en ce que** le filtre de gaz de respiration (82, 82') comporte un rebord imperméable aux gaz, de préférence un cadre imperméable aux gaz (88 ; 88') qui, lorsque le filtre de gaz de respiration (82, 82') est logé prêt à fonctionner dans l'espace de réception du filtre (76 ; 76'), forme une barrière d'écoulement contre un passage de gaz de respiration (74) à travers l'ouverture d'introduction (80 ; 80').

6. Conduit de respiration (65 ; 65') selon l'une des revendications précédentes,
**caractérisé en ce que** la paroi (78 ; 78') fait partie d'un boîtier (77 ; 77') du support de filtre (68 ; 68') entourant l'espace de réception du filtre (76 ; 76') à l'état prêt à fonctionner, dans lequel le boîtier (77 ; 77') présente, en tant que boîtier divisible (77, 77'), au moins deux parties de boîtier (77a', 77b') pouvant être retirées l'une de l'autre et rapprochées l'une de l'autre.

7. Conduit de respiration (65 ; 65') selon l'une des revendications précédentes,
**caractérisé en ce que** le support de filtre (68 ; 68') présente, de part et d'autre de l'espace de réception du filtre (76 ; 76'), des tronçons de conduit (72, 84 ; 72', 84') guidant l'écoulement, dont les tronçons respectivement associés du chemin d'écoulement de gaz de respiration virtuel (AS ; AS') forment entre eux un angle.

8. Conduit de respiration (65) selon l'une des revendications précédentes,
**caractérisé en ce que** le conduit de respiration (65) présente un chauffage de filtre (66) qui entoure au moins par sections le support de filtre (68) à l'état opérationnel.

9. Conduit de respiration (65') selon l'une des revendications précédentes,
**caractérisé en ce que** le filtre de gaz de respiration (82') peut être introduit dans l'espace de réception de filtre (76') le long d'un chemin de déplacement d'introduction (FB'), dans lequel le filtre de gaz de respiration (82') est reçu dans une enveloppe (98') dans un état initial avant son introduction dans l'espace de réception de filtre (76'), dans lequel l'enveloppe (98') présente une formation d'accouplement (102') qui peut être couplée à une contre-formation d'accouplement (104') du support de filtre (68') ou du chauffage de filtre (66) de telle sorte que, dans un état de transfert, dans lequel une ouverture d'accès (106') de l'enveloppe (98') formée pour délivrer le filtre de gaz de respiration (82') à partir de l'enveloppe (98') est alignée avec l'ouverture d'introduction (80') le long du chemin de déplacement d'introduction (FB'), l'enveloppe (98') est maintenue sur la contre-formation d'accouplement (104'), dans lequel au moins une section de paroi (113') de l'enveloppe (98') peut être déplacée par rapport à la formation d'accouplement (102') de telle sorte que le filtre de gaz de respiration (82') peut être extrait de l'enveloppe (98') par l'ouverture d'accès (106') par déplacement de la section de paroi déplaçable (113') par rapport à la formation d'accouplement (102').

10. Conduit de respiration (65') selon la revendication 9,
**caractérisé en ce que** la formation d'accouplement (102') et la contre-formation d'accouplement (104') permettent un déplacement de l'enveloppe (98') par rapport au support de filtre (68') le long d'une trajectoire d'accouplement (KB', KB2') qui s'étend transversalement au chemin de déplacement d'introduction (FB') du filtre de gaz de respiration (82') dans le support de filtre (68').

11. Conduit de respiration (65') selon l'une des revendications 9 ou 10,
**caractérisé en ce qu'**au moins une formation (104') parmi la formation d'accouplement (102') et la contre-formation d'accouplement (104') présente une structure d'étanchéité (124') qui, au moins à l'état de transfert, s'applique de manière étanche contre l'autre formation (102') respective.

12. Conduit de respiration (65') selon l'une des revendications 9 à 11,
**caractérisé en ce que** le filtre de gaz de respiration (82') peut être retiré de l'espace de réception de filtre (76') le long d'un chemin de déplacement de prélèvement (FB'), dans lequel le support de filtre (68') ou le chauffage du filtre (66) présentent une autre contre-formation d'accouplement (120'), dans lequel une enveloppe (98') peut être couplée à l'autre contre-formation d'accouplement (120') de telle sorte que, dans un état de distribution dans lequel l'ouverture d'accès (106') de l'enveloppe (98') est alignée avec l'ouverture de prélèvement (86') le long du chemin de déplacement de prélèvement (FB'), l'enveloppe (98') est maintenue sur l'autre contre-formation d'accouplement (120').

13. Conduit de respiration (65') selon l'une des revendications précédentes,
**caractérisé en ce que** le filtre de gaz de respiration (82') présente un cadre (88') imperméable aux gaz, entourant un matériau filtrant (90'), dans lequel à distance du chemin d'écoulement de gaz de respiration (AS'), de préférence dans la zone de bord de l'ouverture d'introduction (80') ou/et de l'ouverture de prélèvement (86'), de manière particulièrement préférée, en faisant le tour du chemin d'écoulement de gaz de respiration (AS') de manière fermée, il est prévu une formation d'étanchéité (126'), en particulier une lèvre d'étanchéité, qui, dans la position opérationnelle du filtre de gaz de respiration (82'), s'applique de manière étanche contre le cadre (88') du filtre de gaz de respiration (82').

14. Conduit de respiration (65') selon l'une des revendications 1 à 13,
**caractérisé en ce qu'**il présente un agencement de filtre de gaz de respiration (100') comprenant un filtre de gaz de respiration (82') logé dans une enveloppe (98'), dans lequel l'enveloppe (98') présente une formation d'accouplement (102') pour l'accouplement avec une contre-formation d'accouplement (104') d'un support de filtre (68') ou d'un chauffage de filtre (66) ainsi qu'une ouverture d'accès (106') pour la sortie du filtre de gaz de respiration (82 ; 82') hors de l'enveloppe, dans lequel au moins une section de paroi (113') de l'enveloppe (98') peut être déplacée par rapport à la formation d'accouplement (102') de telle sorte que le filtre de gaz de respiration (82') peut être extrait de l'enveloppe (98') par l'ouverture d'accès (106') par déplacement de la section de paroi déplaçable (113') par rapport à la formation d'accouplement (102'), dans lequel l'ouverture d'accès (106') est fermée, à l'état de stockage, avant un accouplement avec le support de filtre (68') ou le chauffage de filtre (66), par une fermeture amovible ou destructible (108').

15. Dispositif de respiration (10) pour la ventilation artificielle au moins partielle d'un patient vivant, comprenant :
- une source de gaz de respiration (12),
- un agencement de conduits de respiration (20) pour conduire le gaz de respiration inspiratoire de la source de gaz de respiration (12) à un orifice de sortie de gaz de respiration proximal (62) côté patient et pour conduire le gaz de respiration expiratoire à l'écart d'un orifice d'entrée de gaz de respiration proximal (62),
- un agencement de capteurs de débit pour la détection quantitative du débit de gaz de respiration inspiratoire ou/et expiratoire dans l'agencement de conduits de respiration (20),
- un dispositif de modification de la pression (13) pour modifier la pression du gaz de respiration dans l'agencement de conduits de respiration (20) ainsi que
- un dispositif de commande (14) pour faire fonctionner la source de gaz de respiration (12) ou/et le dispositif de modification de la pression (13), dans lequel l'agencement de conduits de respiration (20) comprend un conduit de respiration (65 ; 65') selon l'une des revendications précédentes.
